(19) **Europäisches Patentamt**

**European Patent Office**

**Office européen des brevets**

(11) **EP 1 447 451 A1**

(12) **EUROPEAN PATENT APPLICATION**
published in accordance with Art. 158(3) EPC

(43) Date of publication:
**18.08.2004 Bulletin 2004/34**

(51) Int Cl.7: **C12N 15/86**, C12P 21/02,
C07K 14/74, C12N 5/00,
A61K 45/00, A61K 48/00,
A61P 31/00, A61P 35/00

(21) Application number: **02770209.1**

(22) Date of filing: **20.09.2002**

(86) International application number:
**PCT/JP2002/009697**

(87) International publication number:
**WO 2003/029475 (10.04.2003 Gazette 2003/15)**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(30) Priority: **28.09.2001 JP 2001301290**

(71) Applicant: **Dnavec Research Inc.
Tsukuba-shi, Ibaraki 305-0856 (JP)**

(72) Inventors:
• **IWAMOTO, Aikichi
  Bunkyo-ku, Tokyo 113-0022 (JP)**
• **TACHIKAWA, Ai
  Setagaya-ku, Tokyo 154-0016 (JP)**

(74) Representative: **VOSSIUS & PARTNER
Siebertstrasse 4
81675 München (DE)**

(54) **MAMMALIAN CELL-INFECTING VIRUS VECTOR ENCODING EPITOPE-BOUND-BETA2m AND UTILIZATION THEREOF**

(57)    The present inventors constructed mammalian cell-infecting virus vectors encoding epitope-linked-β2m and succeeded in large-scale expression of epitope-linked-β2m in mammalian cells. The present invention provides mammalian cell-infecting virus vectors that encode epitope-linked-β2m microglobulin (β2m) and uses thereof. Furthermore, the present invention provides a method for producing an MHC class I/peptide complex using the vector. In particular, a tetramerized MHC class I/peptide complex is useful in detecting epitope-specific CD8-positive T cells. Moreover, the present invention provides cells introduced with the vector. Target cells added with epitope-linked-β2m produced by the vector of the present invention are also provided. These cells were recognized by antigen-specific cytotoxic T lymphocytes (CTL) . The vectors of this invention and polypeptides obtained via the expression of the vectors are useful in immunotherapy for infections and cancers, and in the detection and quantification of antigen-specific CTL.

EP 1 447 451 A1

**Description**

Technical Field

The present invention relates to mammalian cell-infecting virus vectors encoding epitope-linked-β2m and uses thereof.

Background Art

MHC (major histocompatibility complex) class I molecule is a heterodimer consisting of a heavy chain (HC) and β2-microglobulin (β2m), that is present on the cell membrane surface of almost every cell. MHC class I molecule comprises a groove formed by the heavy chain that can stow a peptide of around 10 amino acids. The resulting MHC class I/peptide complex is recognized through T cell receptors (TCR) on cytotoxic T lymphocytes (CTL) that play a role in cellular immunity. Thus, the peptide displayed on MHC class I molecule is called an "epitope" (antigenic determinant).

Peptide fragments derived from autoantigens produced in the cytoplasm, virus antigens, and tumor antigens are transported into the rough endoplasmic reticulum via a transporter called TAP (transporter associated with antigen processing) existing on the surface of the rough endoplasmic reticulum, and there they form stable MHC class I/peptide complexes with an MHC class I heavy chain and β2m. The β2-microglobulin molecule plays roles in retaining the stability of the 3-dimentional structure of the MHC class I molecule and in the transportation of the heavy chain to the cell surface (Tasuku Honjo and Takeshi Watanabe (ed.), "Immune System:. Recognition, differentiation, and proliferation", The Molecular Biology Society of Japan (ed.), Maruzen Co., Ltd., Tokyo: p117-118). An empty MHC class I molecule unbound to a peptide is known to be very unstable under a culture condition of 37°C.

An epitope, i.e., a peptide displayed on MHC class I molecule, is a very important molecule in inducing specific cellular immunity against a certain antigen. Development of peptide vaccines as immunotherapeutic agents against infections with viruses, such as HIV, or against cancers has been attempted. However, peptides of major epitopes derived from many virus antigens and tumor antigens have weak binding activity to MHC class I molecules. Therefore, generally, when these peptides are used alone as vaccines, no effect can be expected due to their low stability. In 1998, an artificial epitope with amino acid substitutions to enhance the binding ability to MHC class I molecule without losing its antigenicity was effectively used in a clinical study with melanoma patients. However, identification and development of such epitopes with improved stability and antigenicity are troublesome and difficult and not always provide an epitope of interest.

On the other hand, a system using the adjuvant effect of β2m is under development based on the finding that the stability of a peptide on an MHC class I molecule is enhanced by β2m.

Recently, the expression of MHC class I/peptide complexes wherein epitope peptides are fused with MHC class I molecules and MHC class II molecules or β2m has been attempted (Uger, R. A. and Barber, B. H., J. Immunol. 160: 1598-1605 (1998) ; White, J. et al., J. Immunol. 162: 2671-2676 (1999) ;Kang, X. et al., Cancer Res. 57: 202-205 (1997) ; Uger, R. A. et al., J. Immunol. 162: 6024-6028 (1999); USP No. 5,869,270). These complexes are reported to exhibit enhanced stability and recognition by CTL compared to the peptides alone, in particular, those constructed with epitopes having weak binding activity to MHC class I molecules, by the fusion of the epitope peptide sequence via a linker to the N-terminus of β2m (epitope-linked-β2m; e/β2m). However, in these reports, expression vectors for *E. coli* were primarily used, and no report is been known wherein a mammalian cell-infecting virus vector is used. Exogenous proteins overexpressed in *E.coli* often form inclusion bodies and are insolubilized. Although these insoluble proteins can be solubilized with denaturing agents such as urea, and then refolded, not all of these insoluble proteins are dissolved, and furthermore, such treatments are rather troublesome. Moreover, possible contamination of *E.coli* components is another cause of concern.

The efficiency of gene transfer using expression plasmids for mammalian cells is low and sufficient expression cannot be expected. Furthermore, another disadvantage of the use of mammalian cell expression plasmids is that they can be only expressed in dividing cells wherein the nuclear membrane disappears due to the need of the plasmids to be transferred into the nucleus for its expression.

MHC class I/peptide complexes are also useful for assays of antigen-specific CTL. The method so called limiting dilution assay has been used for the determination of frequency of antigen-specific CTL in individuals. However, it requires a long term *in vitro* culture and has a low sensitivity. Accordingly, "MHC class I/peptide tetrameric complex (tetramer)" that is sensitive and can be simply prepared was developed in 1996 (Altman, J. D. et. al., Science 274: 94-96 (1996)). This tetramer is prepared by tetramerization of a monomeric MHC class I/peptide complex using the biotin-avidin binding system followed by the addition of a fluorescence label and is used for FACS analysis. Since this method allows direct analysis of peripheral blood mononuclear cell samples isolated from individuals, it is'not only simple and reflects directly the frequency in each individual, but it also has high sensitivity and high specificity (Wilson, J.D.K. et al., J. Exp. Med. 188: 785-790 (1998); Kuroda, M.J. et al., J. Exp. Med. 187: 1373-1381 (1998)). At present, the MHC class I/peptide complexes that comprise human MHC class I molecules are prepared by independent expression and purification of heavy chains and β2m proteins in *E. coli*, followed by in vitro folding of them with a synthetic peptide.

In contrast, in relation with mouse MHC class I molecules, construction of an insect cell-derived MHC class I/peptide complex using a baculovirus vector has been reported (White, J. et al., J. Immunol. 162: 2671-2676 (1999)). In this system, the MHC class I/peptide complex was constructed in cells co-infected with baculoviruses expressing a heavy chain of MHC class I and an epitope-linked-β2m, respectively. However, there is no report on the use of a mammalian cell-infecting virus vector for this purpose. Since the baculovirus vector system expresses exogenous genes located downstream of a promoter effective in insect cells (for example, Sf9 cells), mammalian cells cannot be utilized (Published Japanese Translation of International Publication No. Hei 6-502990). In addition, while Punnonen et al. have disclosed a genetic vaccine vector for use in DNA shuffling (Punnonen, J. et al., Genetic Vaccine Vector Engineering, USP Application Serial No. 20010006950), a mammalian cell-infecting virus vector encoding epitope-linked-β2m has not been reported.

Disclosure of the Invention

The present invention provides mammalian cell-infecting virus vectors encoding epitope-linked-β2m and uses thereof.

The present inventors intended to construct an expression system using a mammalian cell-infecting virus vector to establish an overexpression system of epitope-linked-β2m and MHC class I/peptide' complex in mammalian cells. For this purpose, the inventors constructed an expression system of Sendai virus (SeV) for the MHC class I/ peptide complex and the epitope-linked-β2m by adopting a recombinant Sendai virus (rSeV) that can be infected into versatile mammalian cells and allows extremely high expression of transgenes.

The inventors used epitopes derived from Nef and Env proteins of HIV as examples for the construction of SeV vector systems that express epitope-linked-β2m. The inventors succeeded in recovering large amounts of the epitope-linked-β2m in the form of secreted protein by introducing the recombinant SeV vectors into mammalian cells. The recovered epitope-linked-β2m was found to form an MHC class I/peptide complex by binding to an MHC class I heavy chain on the cell surface, and had ability to efficiently present the antigen to CTL. Thus, the epitope-linked-β2m produced by the mammalian cell-infecting virus vector was shown to be useful as a protein preparation. Clinically useful antigen-specific cellular immune response-inducing agents can be obtained by recovering and purifying the epitope-linked-β2m produced in cells infected with the vector.

Moreover, the present inventors constructed a SeV vector that expresses an MHC class I heavy chain and used it for co-infection into mammalian cells in combination with a SeV vector expressing an epitope-linked-β2m to recover and purify an MHC class I/peptide complex. Furthermore, using a SeV vector, a secretory MHC class I heavy chain lacking the membrane bound region added with a biotinylation substrate peptide was expressed to yield an MHC class I/peptide complex. This complex was biotinylated, and then an MHC class I/peptide tetramer was constructed via binding the complex through an RPE-labeled avidin. This MHC class I/peptide tetramer is suitably used for the detection and quantification of CTL. This tetramer is glycosylated due to its expression in mammalian cells, and thus is expected to have an increased sensitivity compared with that expressed in an *E.coli* system.

Cells co-infected with SeV vectors expressing HLA-A*2402 and β2m bound to an epitope derived from the HIV-1 Nef protein were found to be recognized by antigen-specific CTL equivalently or more efficiently compared with pulsed administration of a synthetic epitope peptide derived from the HIV-1 Nef protein. Furthermore, when the' effects of epitope-linked-β2m recovered from cells infected with the SeV vector were examined, antigen-specific CTL responses were successfully induced by the addition of epitope-linked-β2m into a culture solution of target cells. These findings indicate that the mammalian cell-infecting virus vector encoding epitope-linked-β2m is useful for *in vivo* or *ex vivo* gene therapy for inducing an antigen-specific immune response. A more efficient immunotherapy than that merely using such peptide vaccines may be achieved by producing a desired MHC class I/peptide complex *in vivo.*

As described above, the inventors established an efficient method for the production of epitope-linked-β2m in mammalian cells, and produced secretory and membrane-bound MHC class I/peptide complexes that contain the epitope-linked-β2m. Furthermore, the inventors developed a novel system for generating an MHC class I/peptide tetramer useful for the detection and quantification of endogenous antigen-specific cytotoxic T lymphocytes (CTL). The inventors further presented an antigen to antigen-specific CTL by binding the epitope-linked-β2m protein prepared by the above described production system to the cell surface MHC class I molecules. Moreover, the inventors established a recognition-inducing system by antigen-specific CTL, through co-infection of target cells with virus vectors expressing epitope-linked-β2m and an MHC class I heavy chain, respectively.

Using the vector system provided by the present invention, a desired epitope-linked-β2m can be expressed in large amounts in mammalian cells. The epitope-linked-β2m and MHC class I/peptide complex thus obtained are quite useful in: (1) detecting and quantifying antigen-specific TLC; (2) displaying antigens on MHC class I molecules using purified epitope-linked-β2m proteins; and (3) inducing antigen-specific cellular immune response via the infection of vectors *in vivo* and *ex vivo*. In particular, the vectors of the present invention are suitably used for the induction of protective immunity against infections with viruses and bacteria, and for immunotherapy against cancers.

The present invention relates to mammalian cell-infecting virus vectors encoding epitope-linked-β2m and uses thereof. More specifically, this invention relates to:

(1) a mammalian cell-infecting virus vector that encodes an epitope-linked-β2m in an expressible manner;

(2) the virus vector of (1), wherein said mammalian cell-infecting virus vector is a Paramyxovirus vector;

(3) the virus vector of (2), wherein said Paramyxovirus is Sendai virus;

(4) the virus vector of any one of (1) to (3), wherein said epitope comprises an amino acid sequence of an epitope peptide presented by an antigen-presenting cell, or a portion thereof;

(5) the virus vector of any one of (1) to (4), wherein said epitope is a partial peptide of a HIV-1 virus protein;

(6) the virus vector of (5), wherein said epitope comprises the amino acid sequence of SEQ ID NO: 24 or 26, or a portion thereof;

(7) the virus vector of any one of (1) to (4), wherein said epitope is a partial peptide of a tumor antigen;

(8) the virus vector of any one of (1) to (7), wherein said vector comprises the amino acid sequence of SEQ ID NO: 13 or a repeated sequence thereof between said epitope and β2m sequences;

(9) a method for producing an epitope-linked-β2m, which comprises the steps of:

(a) introducing the virus vector of any one of (1) to (8) into a mammalian cell, and
(b) recovering the epitope-linked-β2m from the mammalian cell introduced with the vector or the culture supernatant thereof;

(10) an epitope-linked-β2m produced by the method of (9);

(11) a method for producing a heterodimer comprising an epitope-linked-β2m, which comprises the steps of:

(a) introducing the virus vector of any one of (1) to (8) into a mammalian cell, and
(b) recovering the produced heterodimer from the mammalian cell introduced with the vector or the culture supernatant thereof;

(12) a method for producing an MHC class I/peptide complex comprising an MHC class I heavy chain and an epitope-linked-β2m, which comprises the steps of:

(a) introducing the virus vector of any one of (1) to (8) into a mammalian cell, and
(b) recovering the produced MHC class I/peptide complex from the mammalian cell introduced with the vector or the culture supernatant thereof;

(13) the method of (12), wherein said method further comprises the step of introducing a virus vector expressing the MHC class I heavy chain into the mammalian cell;

(14) the method of (13), wherein said MHC class I heavy chain is a secretory form;

(15) the method of (14), wherein said MHC class I heavy chain comprises a biotinylation substrate peptide;

(16) the method of any one of (13) to (15), wherein said MHC class I heavy chain is an A24-restricted HLA class I heavy chain;

(17) the method of (16), wherein said A24-restricted HLA class I heavy chain is derived from A*2402;

(18) the method of (15), wherein said method further comprises the steps of biotinylating said MHC class I heavy chain comprising the biotinylation substrate peptide and assembling the biotinylated MHC class I heavy chain in the presence of avidin;

(19) an MHC class I/peptide complex produced by the method of any one of (12) to (18);

(20) the MHC class I/peptide complex of (19), wherein said complex is a tetramer;

(21) an inducer of antigen-specific cellular immune response comprising the virus vector of any one of (1) to (8), the epitope-linked-β2m of (10), or the MHC class I/peptide complex of (19) or (20) ;

(22) a pharmaceutical composition comprising the virus vector of any one of (1) to (8), the epitope-linked-β2m of (10), or the MHC class I/peptide complex of (19) or (20);

(23) a mammalian cell, wherein the virus vector of any one of (1) to (8) has been introduced;

(24) a cell that has the epitope-linked-β2m of (10) on the cell surface;

(25) the cell of (23) or (24), wherein a gene encoding an MHC class I heavy chain has been exogenously introduced;

(26) the cell of (25), wherein said MHC class I heavy chain is a membrane-bound form;

(27) the cell of (25), wherein said MHC class I heavy chain is a secretory form;

(28) the cell of any one of (23) to (27), which is a dendritic cell;

(29) an inducer of antigen-specific cellular immune response comprising the cell of any one of (23) to (28);

(30) a pharmaceutical composition comprising the cell of any one of (23) to (28); and

(31) a detection agent for antigen-specific T lymphocytes comprising the MHC class I/peptide complex of (19) or (20).

According to the present invention, the term "epitope" refers to a peptide recognized by immune cells. Such immune cells include T cells, B cells, NK cells, and NKT cells. A preferable embodiment of the epitope in this invention includes a peptide recognized by T cells. The epitopes of the present invention include not only those presented by antigen-presenting cells, but may be any desired peptide so long as it is recognized by an immune cell. For example, a peptide having an artificially prepared amino acid sequence may also be used as the epitope. More preferably, the epitope of the present invention is a peptide presented by an antigen-presenting cell (APC) or a portion thereof. In the present invention, a "portion" of a protein or peptide to be used as an epitope contains generally 8 or more, preferably 9 or more, more preferably 10 or more, 12 or more, or 15 or more continuous amino acids.

The present invention provides a mammalian cell-infecting virus vector that encodes an epitope-linked-$\beta$2m. There is no limitation on the mammalian cell-infecting virus vector and any desired virus vector may be used. In addition, the mammalian cell-infecting virus vector of the present invention may have an ability to infect cells other than mammalian cells. Preferably, the vector is less toxic to host cells and achieves a high expression level of a transgenes. The virus vectors used in this invention include, for example, adenovirus vectors, adeno-associated virus vectors, herpes simplex virus vectors, retrovirus vectors, lentivirus vectors, Semliki forest virus vectors, Sindvis virus vectors, vaccinia virus vectors, fowl pox virus vectors, and Sendai virus vectors, but are not limited thereto. Using these vector systems, a recombinant virus vector expressing the epitope-linked-$\beta$2m may be constructed. The phrase "recombinant virus vector" used herein refers to a virus vector generated via a recombinant polynucleotide. The recombinant polynucleotide is defined as a polynucleotide that is not bound as in natural state. More specifically, it refers to artificially recombined polynucleotide chains or newly generated polynucleotides. The "recombinant" virus vector includes, for example, those constructed by gene manipulation and that prepared by gene amplification of the constructed vectors. The recombinant virus vectors may be generated by reconstituting virus particles through the expression of recombinant virus cDNA in host cells.

The recombinant virus vectors may be prepared according to methods known to those skilled in the art. For example, an adenovirus vector that is most frequently used for gene therapy can be constructed according to the method of Saito et al. (Miyake et al., Proc. Natl. Acad. Sci. USA 93: 1320-24 (1996) ; Kanegae et al., "Biomanual Series 4- Gene Transfer and Expression, Methods of Analysis" (in Japanese), 43-58 (1994), Yodosha). For example, a 42 kb cosmid pAdexlcw (Kanegae et al., Saiboukougaku, 13 (8): 757-763 (1994)) that contains a 31 kb Ad DNA, almost the full-length adenovirus (Ad) type 5 genome except for E1 and E3 regions, is cleaved with an appropriate restriction enzyme (for example, *Swa*I), and then properly desalted and purified by gel filtration. An expression unit for exogenous genes is then used for a ligation reaction with the cleaved cosmid using T4 DNA ligase, and the enzyme is inactivated by heat treatment. The resulting ligate is desalted by gel filtration, and then cleaved with *Swa*I. After phenol treatment and gel filtration, the cleaved product is subjected again to *Swa*I cleavage. A portion of the product is used for *in vitro* packaging using Gigapack XL (Stratagene, USA). The ligated product is then introduced into *E.coli* cells. Cosmids are prepared from some of ampicillin-resistant transformants of the cells, their structures are analyzed by digestion with restriction enzymes to isolate recombinant cosmids wherein the exogenous gene expression unit is inserted in the desired direction (opposite direction to that of the transcription of E1A and E1B). In another series, a DNA-terminal protein complex (DNA-TPC) of adenovirus type 5 (Ad5 strain d1X) that lacks E1 and E3 regions is prepared by the method of Saito et al. (Kanegae et al., supra). This complex is digested, for example, with *Eco*T22I, and then desalted and purified by gel filtration and such. The *Eco*T22I-digested fragments are mixed with the recombinant cosmid obtained as described above, and then introduced into 293 cells using, for example, Cellphect kit (Pharmacia, Sweden) . On the next day, the transfected 293 cells are suspended, and the suspension and 10-fold to 100-fold dilution thereof are inoculated onto 96-well plates. Approximately 2 weeks after, a culture solution containing dead cells is isolated from the wells wherein propagation of recombinant Adenovirus (generated via the recombination in the 293 cells) could be observed. The culture solution is repeatedly frozen and thawed to release the adenovirus particles from cells. After centrifugation, the supernatant (primary virus solution) is used for infection of 293 cells spread onto 24-well plates. Three days later, culture solution containing dead cells is isolated. A portion of the solution is frozen and thawed, and centrifuged as for the preparation of the primary virus solution to obtain the supernatant (secondary virus solution). The residual solution is centrifuged to obtain cells. DNA is prepared from the cells and the structure of the recombinant adenovirus DNA is analyzed by digestion with restriction enzymes to select clones that have been confirmed to have the desired DNA structure. The secondary virus solution is used for infection of 293 cells in larger amounts, the culture solution is similarly frozen, thawed and centrifuged to obtain the third virus solution. After determining the titer of the third virus solution according to the method of Saito et al. (Kanegae et al., supra), it may be used as the vector (Miyake et al., supra; Kanegae et al., Acta Paediatr. Jpn, 38: 182-188 (1996)).

In addition, for example, retrovirus vectors (Wakimoto et al., Protein Nucleic Acid and Enzyme 40: 2508-2513 (1995)) and adeno-associated virus vectors (Tamaki et al., Protein Nucleic Acid and Enzyme 40: 2532-2538 (1995)) may also be used. Methods to efficiently produce these vectors are known in the art.

In the interest of producing other vectors that can be used for gene transfer into mammalian cells, Published Japanese Translation of International Publication No. Hei 6-502069, Examined Published Japanese Patent Application No. (JP-B) Hei 6-95937, and JP-B Hei 6-71429 disclose detailed methods for producing recombinant vaccinia viruses. Furthermore, JP-B Hei 6-34727 and Published Japanese Translation of International Publication No. Hei 6-505626 disclose methods for producing recombinant papilloma viruses. Moreover, Unexamined Published Japanese Patent Application No. (JP-A) Hei 5-308975 discloses a method for producing recombinant adeno-associated virus, and Published Japanese Translation of International Publication No. Hei 6-508039 discloses a method for producing recombinant adenovirus.

By introducing the obtained virus vectors into mammalian cells, an epitope-linked-$\beta$2m is expressed and obtained either from the cells or the culture supernatant thereof. The recovered epitope-linked-$\beta$2m is useful for the induction of antigen-specific cellular immune response. For example, an epitope may be presented by adding the epitope-linked-$\beta$2m to antigen-presenting cells. Alternatively, a desired epitope can be efficiently presented by introducing the virus vector expressing the epitope-linked-$\beta$2m into antigen-presenting cells. The epitope may be connected with any site of $\beta$2m, but preferably, for example, it is connected to the N-terminus of the secreted $\beta$2m. For this purpose, the epitope is connected downstream of the secretory signal sequence of $\beta$2m, and the $\beta$2m protein is linked to the epitope, if necessary via a spacer sequence, to yield a fusion protein. There is no limitation on the $\beta$2m to be used, and any desired mammalian $\beta$2m may be used. However, when used for humans, human $\beta$2m is preferred. The human $\beta$2m gene may be isolated according to the method as described in the Examples below.

Furthermore, when both genes encoding epitope-linked-$\beta$2m and a secretory form (also referred to as soluble form) MHC class I heavy chain are introduced into the same mammalian cell, then the MHC class I/peptide complex is secreted into the culture supernatant, allowing easy recovery of the complex. The secretory form MHC class I heavy chain may be introduced into mammalian cells through a virus vector or may be expressed via integration of its gene into the cellular chromosomal DNA. When a biotinylation substrate peptide is attached to the secretory form MHC class I heavy chain, a tetramer of the biotinylated MHC class I/peptide complex can be produced using avidin. Such a tetramer is in particular useful for the detection and quantification of CTL.

When both genes encoding the epitope-linked-$\beta$2m and a membrane-bound form MHC class I heavy chain are introduced into a mammalian cell, a large amount of MHC class I/peptide complex is expressed on the cell surface. Such a cell has an excellent antigen-presenting ability to the epitope-specific CTL.

When expressing an MHC class I heavy chain using a virus vector, the vector is not limited in any way and any desired virus vector may be used similarly as to the virus vectors expressing epitope-linked-$\beta$2m as described above. Alternatively, the epitope-linked-$\beta$2m and the MHC class I heavy chain may be co-expressed from the same vector by inserting the gene encoding the MHC class I heavy chain into a virus vector that expresses the epitope-linked-$\beta$2m. The vector of this invention includes such a co-expression vector that expresses other exogenous genes in addition to the epitope-linked-$\beta$2m. There is no limitation on the MHC class I heavy chain, but human MHC class I (HLA) is preferred when applied to humans. In the Examples, the inventors used A24-restricted HLA class I heavy chain as the MHC class I molecule and a HIV-1-derived epitope presented by the A24-restricted HLA class I molecule as an epitope. Human MHC class I molecules are called human leukocyte antigens (HLA) and are very rich in variety. Among them, about 70% of Japanese have the genotype HLA-A*2402. In the present invention, the A24-restricted HLA class I heavy chain, in particular, HLA-A*2402 is preferred as the MHC class I heavy chain when used for Japanese. A tailor-made medicine is expected to be attained though the selection of an epitope to be bound to $\beta$2m and/or MHC class I molecule that matches the patient's MHC type.

The present inventors also discovered that the use of a mammalian' cell-infecting virus vector enables extremely efficient production of desired secretory form multimeric protein complexes in cells. In particular, secretory forms of homo- or hetero dimers or trimers can be produced with high efficiency using the mammalian cell-infecting virus vectors according to the present invention. For example, even a heteromer protein that is endogenously present on the cell membrane can be secreted extracellularly by expressing it as a protein lacking the transmembrane domain using the mammalian-cell infecting virus vectors as described in the Examples. When producing secretory multimers, such as dimers and trimers, according to the present invention, component proteins of the multimers may be expressed using the mammalian-cell infecting virus vectors. All of these component proteins may be expressed using the mammalian cell-infecting virus vectors, or when the endogenous expression level of some of the components is high enough, only a part of the component proteins such as those with low endogenous expression levels, may be selectively expressed using the mammalian cell-infecting virus vectors. One of the most preferable secretory multimers to be expressed according to this method includes the MHC class II complex. This complex is a double-stranded heterodimer consisting of $\alpha$ and $\beta$ heavy chains. One of the chains may be fused with an epitope to produce a complex similarly to the above-described method used for the production of the epitope-linked-β2m or the epitope-bound MHC class I heavy chain. Another preferable candidate for a secretory form multimer to be expressed using the present method includes T cell receptors. Other candidates include, for example, various cytokines and chemokines that form hetero- or homo-dimers or trimers. Specific examples include heterodimers or trimers such as IL-2, IL-4, IL-7, IL-9, IL-15, IL-3, IL-5, GM-CSF, and homodimers like NK receptors, but are not limited thereto. More specifically, the present invention also provides:

(1) a mammalian cell-infecting virus vector that encodes a component of a secretory multimer in an expressible manner;
(2) the virus vector of (1), wherein said vector is a Paramyxovirus vector;
(3) the virus vector of (2), wherein said Paramyxovirus is Sendai virus;
(4) the virus vector of any one of (1) to (3), wherein said secretory multimer is a secretory homo- or heterodimer, or a secretory homo- or heterotrimer;
(5) the virus vector of (4), wherein said secretory multimer is selected from the group consisting of MHC class II complex, T cell receptor, NK receptor, IL-2, IL-4, IL-7, IL-9, IL-15, IL-3, IL-5, and GM-CSF;
(6) the virus vector of (5), wherein said secretory multimer is an epitope-linked MHC class II complex;
(7) a method for producing a secretory multimer, which comprises the steps of:

(a) introducing the virus vector of any one of (1) to (6) into a mammalian cell, and
(b) recovering the secretory multimer from the mammalian cell introduced with the vector or the culture supernatant thereof;

(8) a secretory multimer produced by the method of (7); and
(9) a mammalian cell introduced with the virus vector of any one of (1) to (6).

In the present invention, particularly preferred mammalian cell-infecting virus vectors include Paramyxovirus vectors. Herein, the phrase "Paramyxovirus vectors" refers to vectors (or carriers) derived from Paramyxovirus and that transfer genes into host cells. Sendai virus (SeV), which belongs to the family *Pramyxoviridae*, has currently been developed as a gene transfer vector (Kato, A. et al., EMBO J. 16: 578-589 (1997) ; WO 97/16538; WO 97/16539). The SeV vector is less toxic and expresses extremely high amounts of proteins from a transferred gene. Furthermore, since genes inserted into the vector are not introduced into the host chromosomes, the SeV vector has an advantage in safety. The SeV vector has a high genome stability, and according to a heterogeneous gene expression experiment, it is shown to stably express the inserted heterogeneous gene for a long time with little mutations in the bases of the gene in spite of continuous multiple passages (Yu, D. et al. , Genes Cells 2, 457-466 (1997)). In addition, the size of the gene that can be introduced and the flexibility of packaging due to the lack of capsid structure protein is another advantageous property of the SeV vector. The SeV vector with replication ability allows introduction of an exogenous gene of at least up to 4 kb. In addition, two or more genes may be simultaneously expressed by adding transcription units to the vector. Virus vectors replicated from a vector based on the replicon of SeV reinfect into adjacent cells. Then, multiple copies of RNP replicated in the cytoplasm of the infected cells distribute over daughter cells during cell division, and thus the vector is expected to continuously express. Moreover, the SeV vector has a wide tissue adaptability.

Since Paramyxovirus vectors are not pathogenic in humans, they can be suggested to be preferably utilized in clinical trials of human gene therapy in view of safety. It is a major obstacle in high efficient gene transfer that, in most cases, introduced DNA must be transported into the nucleus or nuclear membrane must be eliminated for the expression of a transgene via plasmid DNA or such. In the case of Sendai virus, however, expression of an exogenous gene is driven by both cellular tubulin and its RNA polymerase (L protein) in the cytoplasm when virus genomes replicate. This suggests that the Sendai virus does not interact with chromosomes of host cells, which avoids risks such as cancerization and immortalization of cells. Furthermore, the Sendai virus is known to be pathogenic in rodents causing pneumonia, but not in humans, which is supported by studies showing that the intranasal administration of the wild-type Sendai virus does not do harm in nonhuman primates (Hurwitz, J. L. et al., Vaccine, 15: 533-540 (1997)). These features suggest that Sendai virus vector can be utilized in human therapy, and further support the notion that Sendai virus vectors can be one of the promising tools for gene therapy with the aim of *in vivo* or *ex vivo* expression of epitope-linked β2m.

The Paramyxovirus vector of the present invention may be ribonucleoprotein (RNP) or a virus particle possessing infectivity. Herein, the term "infectivity" refers to an ability of the recombinant Paramyxovirus vector to transfer a gene contained in the vector into cells to which the vector adheres due to its cell adhesion and membrane fusion abilities. The Paramyxovirus vector of the present invention may have replication ability, or may be a defective vector without the replication ability. Herein, "replication ability" is defined as the ability of virus vectors to replicate and produce infective virus particles in host cells infected with the virus vectors. Host cells include, for example, LLC-MK2 and CV-1. Details of the present invention are described below using the Paramyxovirus vector as an example, however, the virus vectors of the present invention are not limited thereto, and further include other virus vectors that can be constructed based on the description below.

Herein, the term "Paramyxovirus" is defined as a virus of the *Paramyxoviridae* family or a derivative thereof. Paramyxoviruses used in the present invention include, for example, viruses belonging to the *Paramyxoviridae* such as Sendai virus, Newcastle disease virus, Mumps virus, Measles virus, Respiratory syncytial virus, rinderpest virus, distemper virus, simian parainfluenza virus (SV5), and type I, II, and III human parainfluenza virus. Preferred virus of the present invention include those belonging to the subfamily *Paramyxoviridae* (including the genera of Respirovirus, Rubulavirus, and Mobillivirus), more preferably the genus Respirovirus (also called Paramyxovirus) or a derivative thereof. Respiroviruses that can be used in the present invention include, for example, type I human parainfluenza virus (HPIV-1), type III human parainfluenza virus (HPIV-3), type III bovine parainfluenza virus (BPIV-3), Sendai virus (also called type I mouse parainfluenza virus), and type X simian parainfluenza virus (SPIV-10) . Most preferable Paramyxovirus of the invention is Sendai virus. These viruses may be naturally occurring, wild-type, mutant, laboratory-passaged, artificially constructed strains, etc. Incomplete viruses such as the DI particle (Willenbrink, W. and Neubert, W.J., J. Virol. 68: 8413-8417 (1994)) and synthesized oligonucleotides may be utilized as a material for generating the virus vector of the present invention.

Genes encoding proteins of a Paramyxovirus include NP, P, M, F, HN, and L genes. Herein, the "NP, P, M, F, HN, and L genes" represent those encoding the nucleocapsid protein, phosphoprotein, matrix protein, fusion protein, hemagglutinin-neuraminidase, and large protein, respectively. Genes of each virus of the subfamily Paramyxovirus are described generally as follows. In general, NP gene may also be indicated as "N gene".

| Respirovirus | NP | P/C/V | M | F | HN | - | L |
| Rublavirus | NP | P/V | M | F | HN | (SH) | L |
| Morbillivirus | NP | P/C/V | M | F | H | - | L |

For instance, the accession numbers of each gene of the Sendai virus classified as a Respirovirus genus of *Paramyxoviridae* in the nucleotide sequence database, are M29343, M30202, M30203, M30204, M51331, M55565, M69046, and X17218 for NP gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. As used herein, the term "gene" refers to a genetic substance, including nucleic acids such as RNA and DNA. There is no restriction as to the origin of genes and it can be a naturally-occurring sequence or an artificially designed sequence. Furthermore, as used herein, the term "DNA" includes single-stranded DNA and double-stranded DNA.

Paramyxovirus vectors used in the present invention is not particularly limited. For instance, preferable Paramyxovirus vectors include vectors that are able to replicate and autonomously proliferate. In general, for example, the genome of wild type Paramyxoviruses contain a short 3' leader region followed by six genes encoding nucleocapsid (N), phospho (P), matrix (M), fusion (F), hemagglutinin-neuraminidase (HN), and large (L) proteins, and has a short 5' trailer region on the other terminus. Vectors of the present invention that are able to replicate autonomously can be obtained by designing a genome having a similar structure to that as described above. In addition, a vector for expressing a gene encoding epitope-linked $\beta 2m$ and/or MHC class I heavy chain can be obtained by inserting the gene to the genome of the above vector. Paramyxovirus vectors of the invention may have an altered alignment of virus genes on the vector, compared with wild type viruses.

Paramyxovirus vectors of the present invention may have any deletion of the genes that are contained in the wild-type Paramyxovirus. For instance, when Sendai virus vectors are reconstituted, proteins encoded by NP, P/C, and L genes are thought to be required in trans, but the genes themselves may not be a component of virus vectors of the present invention. For example, an expression vector carrying genes encoding the proteins may be co-transfected into host cells with another expression vector encoding the vector genome to reconstitute a vector. Alternatively, an expression vector encoding the virus genome is introduced into host cells carrying genes encoding the proteins, and then the vector can be reconstituted by using the proteins derived from the host cell. The amino acid sequence of these proteins may not be identical to those derived from the original virus as long as it has an equivalent or higher activity in nucleic acid transfer, and may be mutated or replaced with that of a homologous gene of another virus.

Proteins encoded by M, F, and HN genes are thought to be essential for cell-to-cell propagation of a Paramyxovirus vector. However, these proteins are not required when a Paramyxovirus vector is prepared as RNP. If genes M, F, and HN are components of the genome contained in RNP, products of these genes are produced when introduced into host cells, and virus particles having infectivity are generated. RNP vectors that produce an infective virus include, for example, a viral genomic RNA encoding N, P, M, F, HN, and L genes and N, P, and L proteins. When such RNP is introduced into cells, virus genome is expressed and replicated through functions of N, P, and L proteins, and thus infective virus vectors are amplified. Thus, the Paramyxovirus vector can be amplified by introducing the RNP that is generated during the reconstitution process of the Paramyxovirus vector and such into host cells, such as LLC-MK2 cells, and culturing the introduced cells. This amplification includes the steps of: (a) introducing a negative single-stranded RNA derived from Paramyxovirus and a complex consisting of NP, P/C, and L-proteins into a cell, and (b) culturing the introduced cell and recovering the virus particles from the culture supernatant thereof.

RNP can be introduced into cells as a complex with, for example, lipofectamine and polycationic liposome. Specifically, a variety of transfection reagents can be used, for instance, DOTMA (Boehringer), Superfect (QIAGEN #301305), DOTAP, DOPE, and DOSPER (Boehringer #1811169). Chloroquine may be added to prevent degradation in the endosome (Calos, M.P., Proc. Natl. Acad. Sci. USA 80: 3015 (1983)). For replicative viruses, the produced viruses can be amplified or passaged by re-infecting into cultured cells, chicken eggs, or animals (e.g., mammalian such as mice).

Paramyxovirus vectors lacking the M, F, and/or HN genes are also used as Paramyxovirus vectors of the present invention. These virus vectors can be reconstituted by providing deleted gene products exogenously. Such vectors can still adhere to host cells and induce cell fusion like the wild-type virus. However, daughter virus particles do not have the same infectivity as the parent ones because the vector genome introduced into cells lacks one or more of these genes. Therefore, these vectors are useful as safe virus vectors that are capable of only a single gene transfer. For example, genes deleted from the genome may be F and/or HN genes. Virus vectors can be reconstituted by co-transfection of an expression plasmid encoding the genome of a recombinant Paramyxovirus lacking the F gene, with an expression vector for the F protein, and that for NP, P/C, and L proteins into host cells (International Publication numbers WO 00/70055 and WO 00/70070). Alternatively, host cells in which the F gene is integrated into the chromosome may be used. The amino acid sequence of these proteins provided exogenously may not be identical to those of the wild-type and may be mutated or replaced by a homologous protein of another virus as long as they provide equivalent or higher gene transfer activity.

The envelope protein of the virus vectors of the invention may contain a protein other than the envelope protein of the original vector genome. For example, virus vectors having desired envelope proteins can be produced by intracellularly expressing envelope protein other than that encoded by the virus genome during viral reconstitution. There is no limitation on such proteins. These include envelope proteins of other viruses such as the G protein (VSV-G) of the vesicular stomatitis virus (VSV). Thus, virus vectors of the invention include a pseudo-type virus vector that has an envelope protein derived from a virus different from the original virus.

Viral vectors of the present invention may also comprise, for example, on the viral envelop surface, proteins capable of adhering to particular cells, such as adhesion factors, ligands and receptors or chimeric proteins comprising a protein described above on the outer surface and viral envelop-derived polypeptides inside the virus. It enables the production of a vector targeting a particular tissue. These proteins may be encoded by the virus genome itself, or supplied at the time of virus reconstitution through expression of genes other than virus genome (for example, genes derived from another expression vector or host cell chromosome).

The virus genes contained in the vector of the present invention may be altered, for example, to reduce antigenicity of the virus protein derived from the vector, or enhance RNA transcription efficiency or replication efficiency. Specifically, in the Paramyxovirus vector, it is possible to alter at least one of the NP, P/C, and L genes, which are genes of replication factors, to enhance transcription or replication. It is also possible to alter the HN protein, a structural protein having hemagglutinin activity and neuraminidase activity, to enhance the virus stability in blood by weakening the former activity and to regulate infectivity by altering the latter activity. It is also possible to alter the F protein, which is implicated in membrane fusion, to regulate its fusion ability of the membrane-fused liposome. Furthermore, it is possible to analyze the antigen presenting epitopes and such of possible antigenic molecules on the cell surface, such as the F protein and HN protein, and use them to generate a Paramyxovirus vector that is engineered to have weak ability to present these proteins as antigens.

Paramyxovirus vectors of the present invention may also lack accessory genes. For example, the disruption of the V gene, one of the accessory genes of SeV, results in reduction of pathogenicity of SeV toward hosts such as mouse without affecting gene expression and replication in cultured cells (Kato, A. et al., J. Virol. 71:7266-7272 (1997); Kato, A. et al., EMBO J. 16: 578-587 (1997); Curran, J. et al., WO 01/04272, EP 1067179). Such attenuated vectors are particularly suitable as Paramyxovirus vectors of the present invention.

Recombinant Paramyxovirus vectors bearing exogenous genes, such as those encoding epitope-linked-β2m or MHC class I heavy chain, can be obtained by inserting these genes into the above-described Paramyxovirus vector genome. The exogenous gene encoding the β2m and the MHC class I heavy chain may be genes encoding naturally-occurring proteins. Alternatively, these may be genes encoding modified proteins with deletion, substitution or insertion relative to the naturally-occurring protein so long as the modified protein is functionally equivalent to the naturally-occurring protein, so long as the modified protein has an ability to form an MHC class I/peptide complex, or the formed complex is recognized by cytotoxic T cells or activates CTL. In the case of inserting an exogenous gene into virus vector DNA, such as Sendai virus vector DNA, a sequence comprising nucleotides of multiples of six is desirably inserted between the transcription end sequence (E) and the transcription start sequence (S) (J. Virol., 67 (8) : 4822-4830 (1993)). An exogenous gene can be inserted upstream and/or downstream of each of the virus genes (NP, P, M, F, HN, and L genes). In order not to interfere with the expression of upstream and downstream genes, an E-I-S sequence (transcription end sequence-intervening sequence-transcription start sequence) or a portion of it may be suitably placed upstream or downstream of an exogenous gene so that the unit of E-I-S sequence is located between each gene. Alternatively, an exogenous gene can be inserted via IRES sequence.

Expression level of inserted exogenous genes can be regulated by the type of transcription start sequence that is attached to the upstream of the genes (WO 01/18223). It also can be regulated by the position of insertion and the sequence surrounding the gene. In the Sendai virus, for instance, the closer to the 3'-terminus of the negative strand RNA of the virus genome (the closer to NP gene in the gene arrangement on the wild-type virus genome) the insertion position is, the higher the expression level of the inserted gene will be. To achieve a high expression of an inserted gene, it is preferably inserted into the upstream region (3' flanking sequence of the negative strand genome) such as the upstream of the NP gene (3' flanking sequence on the negative strand), or between NP and P genes. Conversely, the closer to the 5'-terminus of the negative strand RNA (the closer to L gene in the gene arrangement on the wild-type virus genome) the insertion position is, the lower the expression level of the inserted gene will be. To reduce the expression of an exogenous gene, it may be inserted into the most 5' position on the negative strand, that is, downstream of the L gene in the wild-type virus genome (5' flanking region of the L gene on. the negative strand) or upstream of the L gene (3' flanking region of L gene on the negative strand). Thus, the insertion position of an exogenous gene can be properly adjusted to obtain a desired expression level of the gene or optimize the combination of the insert with the virus genes surrounding it. For instance, if the overexpression of a transgene introduced by a high-titer virus vector may cause toxicity, it is possible not only to control the titer of viruses to be administered but also to reduce the expression level of individual virus vectors by designing the insertion position of the transgene closer to the 5'-terminus of the negative strand, or replacing the transcription start sequence with one having lower efficiency so as to obtain an appropriate expression level or therapeutic effect.

Generally, since high level expression of epitope-linked-β2m is thought to be advantageous for efficient production of the protein and induction of immunity as long as it shows no cytotoxicity, it is preferred to bind the gene encoding the epitope-linked-β2m to a highly efficient transcription start sequence and insert it adjacent to the 3'-terminus of the negative strand genome. Preferred vectors include those wherein the gene encoding the epitope-linked-β2m is placed in the 3' flanking region of all of the Paramyxovirus protein genes on the negative strand genome. For example, a vector in which the exogenous gene is inserted upstream of the N gene (in the 3' flanking region of the coding sequence of the N gene on the negative strand) is preferred. Alternatively, it may be inserted immediately downstream of the N gene.

To help the easy insertion of an exogenous gene, a cloning site may be designed at the position of insertion in the vector DNA encoding the genome. For example, the cloning site may be the recognition sequence of a restriction enzyme. The cloning site may be a multicloning site that contains recognition sequences for multiple restriction enzymes. The vector of the present invention may have other exogenous genes at positions other than that used for the insertion of the epitope-linked β2m gene. Such exogenous gene may be, without limitation, a chemokine or cytokine gene, MHC class I heavy chain gene, or another gene.

Construction of a recombinant Sendai virus vector having an exogenous gene can be performed as follows, for example, according to the method described in Hasan, M.K. et al., J. Gen. Virol. 78: 2813-2820 (1997); Kato, A. et al., EMBO J. 16: 578-587 (1997); and Yu, D. et al., Genes Cells 2: 457-466 (1997).

First, a DNA sample containing a cDNA nucleotide sequence encoding a desired exogenous gene is prepared. It is preferable that the concentration of the DNA sample is 25 ng/μl or higher and that it can be detected as a single plasmid by electrophoresis. The following description is an example where an exogenous gene is inserted into the *Not*I site of virus genomic DNA. When the target cDNA sequence contains a *Not*I recognition site, the site is desirably removed in advance by altering the nucleotide sequence using known methods, such as site-directed mutagenesis, while maintaining the encoded amino acid sequence. A desired DNA fragment is amplified by PCR from a DNA sample. In order to obtain a fragment having the *Not*I site at both ends and to add a single copy of the transcription end sequence (E), intervening sequence (I), and transcription start sequence (S) of the Sendai virus (EIS sequence) to one end, synthesized DNA sequences (primer pair), namely, a pair of a forward primer (sense strand) comprising a part of the desired gene, and a reverse primer (antisense) comprising a *Not*I recognition site, E, I, and S sequences, and part of the desired gene, is prepared.

For example, the forward synthetic DNA sequence contains two or more nucleotides at the 5'-terminus to ensure digestion with *Not*I (preferably 4 nucleotides not containing a sequence derived from the NotI recognition site, such as GCG and GCC; more preferably ACTT). To the 3'-terminus of the sequence, the *Not*I recognition sequence GCGGCCGC is added. Furthermore, to the 3'-terminus, as a spacer, any 9 nucleotides or those of 9 plus multiples of 6 are added. Furthermore, to the 3'-terminus, a sequence of approximately 25 nucleotides corresponding to the ORF of the desired cDNA starting from the initiation codon ATG is added. The 3'-terminus of the forward synthetic oligo DNA containing approximately 25 nucleotides of the desired cDNA is preferably selected so that the last nucleotide is G or C.

The reverse synthetic DNA sequence contains two or more nucleotides at the 5'-terminus (preferably 4 nucleotides not containing a sequence derived from the *Not*I recognition site, such as GCG and GCC; more preferably ACTT). To the 3'-terminus of the sequence, the *Not*I recognition sequence GCGGCCGC. is added. Furthermore, to the 3'-terminus, a spacer oligo DNA is added to adjust the length of the primer. The length of the oligo DNA is designed so that it is a multiple of 6 nucleotides including the *Not*I recognition sequence GCGGCCGC, the sequence complementary to the cDNA, and the EIS sequence derived from the Sendai virus genome as described below (so-called "rule of six"; Kolakofski, D. et al., J. Virol. 72: 891-899 (1998); Calain, P. and Roux, L., J. Virol. 67: 4822-4830 (1993)). Furthermore, to the 3'-terminus of the added sequence, complementary sequences to the S sequence of the Sendai virus, preferably. 5'-CTTTCACCCT-3' (SEQ ID NO: 1) , sequence complementary to the I sequence, preferably 5'-AAG-3', and to the E sequence, preferably 5'-TTTTTCTTACTACGG-3' (SEQ ID NO: 2) are added. Finally, to the 3'-terminus, a sequence, which is selected so that the last nucleotide of the complementary sequence of the desired cDNA becomes G or C, is added, where the last nucleotide is approximately 25 nucleotides upstream from the termination codon. Thus, the 3'-teminus of the reverse synthetic oligo DNA is prepared.

PCR can be performed by a common method using, for example, ExTaq polymerase (TaKaRa). Vent polymerase (NEB) may be used preferably, and the amplified fragment is digested with *Not*I, and inserted into the *Not*I site of plasmid vector pBluescript. The nucleotide sequence of the obtained PCR product is checked with automated DNA sequencer, and a plasmid having the correct sequence is selected. The insert is excised from the plasmid by *Not*I digestion, and subcloned into the *Not*I site of the plasmid comprising Paramyxovirus genomic cDNA. Alternatively, the PCR products may be directly cloned into the *Not*I site of the virus genome cDNA without using a plasmid vector to obtain recombinant Sendai virus cDNA.

For example, recombinant Sendai virus genomic cDNA can be constructed according to the methods described in the literature (Yu, D. et al. , Genes Cells 2: 457-466 (1997) ; Hasan, M.K. et al. , J. Gen. Virol. 78: 2813-2820 (1997) ) . For example, a 18-bp spacer sequence containing the *Not*I site (5'-(G)-CGGCCGCAGATCTTCACG-3'; SEQ ID NO: 3) is inserted into an adjacent gene locus of a cloned Sendai virus genomic cDNA (pSeV (+) ) between the leader sequence and the 5'-terminus of a sequence encoding the N protein, and the plasmid pSeV18+b(+) containing a self-cleavable ribozyme site derived from the antigenomic strand of the hepatitis delta virus is obtained (Hasan, M.K. et al., J. General Virol. 78: 2813-2820 (1997)). An exogenous gene fragment is inserted into the *Not*I site of pSeV18+b (+) to obtain a recombinant Sendai virus cDNA into which a desired exogenous gene has been inserted.

A DNA encoding epitope-linked-β2m is inserted into this vector. There is no limitation on the epitope used in the present invention, and a variety of desired epitopes having therapeutic effects against, for example, tumors, infectious diseases, and other general diseases may be used. Generally, it is preferred to use a peptide of around 10 amino acids as the epitope, but the length of the peptide may be changed appropriately. The epitope-linked-β2m may be, for example, a protein wherein the epitope is linked next to the secretion signal of β2m and β2m is linked to the epitope via a spacer sequence with an appropriate length. Alternatively, the epitope sequence may be directly bound to β2m without a spacer. The amino acid sequence of the spacer is not limited in any way, but preferred peptides used as the spacer include those comprising an amino acid sequence, for example, Gly-Gly-Gly-Ser (SEQ ID NO: 13) or repeated sequences thereof, (Gly-Gly-Gly-Ser)$_n$. The number of repetition (n) is not limited, however it is, for example, 1 to 5 (1, 2, 3, 4, or 5). When using a spacer, it preferably has a length of up to 16 amino acids, for example, 4, 8, or 16 amino acids.

The recombinant Paramyxovirus vector DNA prepared as described above is bound to an appropriate transcription promoter and the resultant DNA is transcribed *in vitro* or intracellularly, and RNP is reconstituted in the presence of viral L, P, and NP proteins to produce a Paramyxovirus vector comprising the RNP. The present invention provides a method for producing a Paramyxovirus vector, the method comprising the steps of transcribing DNA encoding the Paramyxovirus vector genome. The present invention also provides DNA for producing a Paramyxovirus vector for the present invention, wherein said DNA comprises the above-mentioned DNA. The present invention also relates to the use of DNA encoding the vector genome for producing Paramyxovirus vectors of the present invention. Reconstitution of a virus from virus vector DNA can be performed according to known methods (WO 97/16539; WO 97/16538; Durbin, A.P. et al., Virol. 235: 323-332 (1997); Whelan, S.P. et al., Proc. Natl. Acad. Sci. USA 92: 8388-8392 (1995); Schnell, M.J. et al., EMBO J. 13: 4195-4203 (1994) ; Radecke, F. et al. , EMBO J. 14: 5773-5784 (1995) ; Lawson, N.D. et al., Proc. Natl. Acad. Sci. USA 92: 4477-4481 (1995) ; Garcin, D. et al., EMBO J. 14: 6087-6094 (1995); Kato, A. et al., Genes Cells 1: 569-579 (1996) ; Baron, M.D. and Barrett, T. , J. Virology 71: 1265-1271 (1997) ; Bridgen, A. and Elliott, R.M. , Proc. Natl. Acad. Sci. USA 93: 15400-15404 (1996)). These methods enable the reconstitution of desirable Paramyxovirus vectors including the parainfluenza virus, vesicular stomatitis virus, rabies virus, measles virus, rinderpest virus, and Sendai virus vectors and the other (-) strand RNA viral vectors from DNA. If the F, HN, and/or M genes are deleted from the virus vector DNA, infective virus particles will not be formed. However, it is possible to generate infective virus particles by introducing these deleted genes and/or genes encoding an envelope protein from another virus into the host cells and expressing them.

Paramyxovirus vectors are generally prepared by the steps of: (a) transcribing a vector DNA encoding a negative single-stranded RNA or its complementary strand (positive strand) derived from Paramyxovirus in cells (helper cells) expressing the NP, P, and L proteins; and (b) culturing the cells and recovering virus particles from the culture supernatants. The RNA transcribed from the vector DNA forms an RNP complex with the NP, L, and P proteins, leading to the generation of virus particles encapsulated in outer envelope containing envelope proteins.

The DNA encoding the virus genome (vector DNA) that is expressed in the helper cells encodes the minus strand (negative strand RNA) of the virus genome or its complementary strand (positive strand RNA) . For example, the DNA encoding the negative single-stranded RNA or its complementary strand is bound downstream of T7 promoter and then transcribed to RNA with T7 RNA polymerase. Any desired promoter sequence may be used in place of a promoter that includes a sequence recognized by T7 RNA polymerase. Alternatively, an *in vitro* transcribed RNA may be transfected into helper cells. Although both positive and negative strands of the virus genome may be transcribed in the cell, it is preferred to let the positive strand transcribe to enhance the reconstitution efficiency.

Methods for introducing vector DNA into cells include (1) a method for forming DNA precipitates that can be incorporated into desired cells; (2) a method for making a complex that comprises positively charged DNA that is suitable for being incorporated into desired cells and that has low cytotoxicity; and (3) a method for instantaneously opening a pore large enough for DNA to pass through the desired plasma membrane using an electrical pulse.

A variety of transfection reagents can be used in (2), for instance, DOTMA (Boehringer) , Superfect (QIAGEN#301305), DOTAP, DOPE, and DOSPER (Boehringer #1811169). For (1), transfection using calcium phosphate can be used. In this method, DNA incorporated by cells is taken up into phagocytic vesicles, but it is known that a sufficient amount of DNA is also taken up into the nucleus (Graham, F.L. and van Der Eb, J., Virology 52: 456 (1973); Wigler, M. and Silverstein, S., Cell 11: 223 (1977) ) . Chen and Okayama studied the optimization of the transfer technology and reported (1) that maximal efficiency is obtained when cells and precipitates are incubated under 2% to 4% $CO_2$ at 35°C for 15 hr to 24 hr; (2) that circular DNA has higher activity than linear DNA; and (3) that the optimal precipitates are formed when the DNA concentration in the mixed solution is 20 μg/ml to 30 μg/ml (Chen, C. and Okayama, H. , Mol. Cell. Biol. 7: 2745 (1987) ) . The method of (2) is suitable for transient transfection. More classically, a transfection method in which DEAE-dextran (Sigma #D-9885 M. W. 5x $10^5$) is mixed with DNA at a desired concentration ratio is known. Because most complexes are degraded in the endosome, chloroquine may be added to enhance the transfection efficiency (Calos, M.P., Proc. Natl. Acad. Sci. USA 80: 3015 (1983) ) . The method of (3) , called electroporation, may be more broadly applied than the methods of (1) and (2) because it can be used for any kind of cells. The transfection efficiency can be maximized by optimizing the duration of pulse currents, the form of pulse, the strength of the electrical field (gap between electrodes, and voltage) , conductivity of buffer, DNA concentration, and cell density.

Among the above three methods, the method of (2) is easy to perform and enables the testing of a large number of samples using a large amount of cells. Therefore, transfection reagents are suitably used to introduce DNA into cells to reconstitute vectors. Preferable transfection reagents include, Superfect Transfection Reagent (QIAGEN, Cat No. 301305) and DOSPER Liposomal Transfection Reagent (Boehringer Mannheim, Cat No. 1811169), but are not limited thereto.

Specifically, the reconstitution from cDNA can be performed as follows.

LLC-MK2, a cell line derived from a monkey kidney, is cultured on a plastic plate with around 24- to 6-wells, a 100-mm petri dish, or such, in minimum essential medium (MEM) containing 10% fetal calf serum (FCS) and antibiotic (100 units/ml penicillin G and 100 μg/ml streptomycin) to be 70% to 80% confluent. Cells are then infected, for instance, at 2 pfu/cell with recombinant vaccinia virus vTF7-3 that expresses T7 polymerase, which has been inactivated by a 20-minute UV exposure in the presence of 1 μg/ml psoralen (Fuerst, T.R. et al., Proc. Natl. Acad. Sci. USA 83: 8122-8126 (1986); Kato, A. et al., Genes Cells 1: 569-579 (1996)). The amount of psoralen and the duration of UV exposure can be optimized. One hour after infection, cells are transfected by, for example, lipofection using Superfect (QIAGEN) with 2 μg to 60 μg of, or more preferably 3 μg to 5 μg of the above recombinant Sendai virus cDNA together with expression plasmids for virus proteins (24-0.5 μg pGEM-N, 12-0.25 μg pGEM-P, and 24-0.5 μg pGEM-L, or more preferably 1 μg pGEM-N, 0.5 μg pGEM-P, and 1 μg pGEM-L) (Kato, A. et al. , Genes Cells 1: 569-579 (1996)) that function in trans and are required for producing a full-length Sendai virus genome. The transfected cells are cultured in serum-free MEM containing, if desired, 100 μg/ml rifampicin (Sigma) and cytosine arabinoside (AraC) (Sigma), more preferably 40 μg/ml arabinoside alone; the drug concentration is adjusted to be optimal to minimize the cytotoxicity of the vaccinia virus and maximize the recovery of virus (Kato, A. et al., Genes Cells 1: 569-579 (1996)). Cells are cultured for 48 hr to 72 hr after transfection, then collected and lysed through three cycles of freeze-thawing. The cell lysates are transfected into LLC-MK2 cells and the cells are cultured. Alternatively, culture supernatants are recovered and added to the culture solution of LLC-MKK2 cells to infect the cells and then the cells are cultured. After a 3-day to 7-day culture, the culture medium is collected. Alternatively, the above-described freeze-thaw cell lysates are inoculated into the allanto of 10-day old developing chicken eggs, and three days later, allantoic fluid may be recovered. To reconstitute a virus vector lacking a gene encoding an envelope protein that is incapable of replication, the vector may be transfected into LLC-MK2 cells expressing an envelope protein, or co-transfected with expression plasmid for the envelope protein. Alternatively, transfected cells can be overlaid and cultured on LLC-MK2 cells expressing envelope protein to propagate a deletion virus vector (see International Publication Numbers WO 00/70055 and WO 00/70070). The virus titer of the culture supernatant or allantoic fluid can be determined by measuring hemagglutinin activity (HA). The HA may be determined by "end-point dilution" (Kato, A. et al., Genes Cells 1: 569-579 (1996); Yonemitsu, Y. and Kaneda, Y., "Hemagglutinating virus of Japan-liposome-mediated gene delivery to vascular cells.", Molecular Biology of Vascular Diseases. Methods in Molecular Medicine, Ed. by Baker A. H., Humana Press, 295-306 (1999)). To eliminate the possible contamination of vaccinia virus expressing T7 polymerase, the obtained allantoic fluid sample may be diluted appropriately ($10^6$ times for instance) and re-amplified in chicken eggs. Re-amplification may be repeated, for example, three times or more. The obtained virus stock can be stored at -80°C.

Host cells for viral reconstitution are not limited to any special types of cells as long as the virus vector can be reconstituted in the cells. For example, in order to reconstitute a SeV vector and the like, host cells including monkey kidney-derived cells such as LLC-MK2 cells and CV-1 cells, cultured cell lines such as BHK cells derived from a hamster kidney, and human-derived cells may be used. By expressing appropriate envelope proteins in these cells, infectious viral particles that include the proteins in its envelope can be obtained. Furthermore, to obtain a large quantity of the Sendai virus vector, embryonated chicken eggs may be infected with virus vectors obtained from the above host cells and the vectors can be amplified. The method of producing virus vectors using chicken eggs has been established (Advanced protocols in neuroscience study III, Molecular physiology in neuroscience., Ed. by Nakanishi et al., Kouseisha, Osaka, 153-172 (1993)). Specifically, for example, fertilized eggs are incubated for 9 days to 12 days at 37°C to 38°C in an incubator to grow the embryos. Virus vectors are inoculated into the allantoic cavity, and eggs are further incubated for several days to propagate the vectors. Conditions such as the duration of incubation may vary depending on the type of recombinant Sendai virus used. Then, the allantoic fluids containing viruses are recovered. Sendai virus vector is separated and purified from the allantoic fluid sample according to a standard method (Tashiro, M., "Protocols in virus experiments.", Ed. by Nagai and Ishihama, MEDICAL VIEW, 68-73 (1995)).

The construction and the preparation of Sendai virus vectors deficient in F protein can be performed, for example, as follows (see WO00/70055 and WO00/70070).

<1> Construction of F-deficient Sendai virus genome cDNA and F-expression plasmid

Full-length Sendai virus (SeV) genomic cDNA, pSeV18$^+$b(+) (Hasan, M.K. et al., J. Gen. Virol. 78: 2813-2820 (1997)) ("pSeV18$^+$b(+)" is also referred to as "pSeV18$^+$") , is digested with *Sph*I/*Kpn*I and the digested fragment (14673 bp) is recovered. The fragment is subcloned into pUC18 to obtain plasmid pUC18/KS. Construction of F gene-deficient region is performed using this pUC18/KS with a combination of PCR and ligation techniques. F gene-deficient SeV genomic cDNA (pSeV18$^+$/ΔF) is constructed by removing the F gene ORF (ATG-TGA= 1698 bp) and filling in the gap with, for example, atgcatgccggcagatga (SEQ ID NO: 4). PCR products obtained by primer pairs consisting of forward: 5'-gttgagtactgcaagagc (SEQ ID NO: 5) and reverse: 5'-tttgccggcatgcatgtttcccaaggggagagtttgcaacc (SEQ ID NO: 6), and primer pairs consisting of forward: 5'- atgcatgccggcagatga (SEQ ID NO:7) and reverse: 5' - tgggtgaatgagagaatcagc (S EQIDNO: 8) are linked at *Eco*T22I to the upstream and downstream of F gene, respectively. The thus-obtained plasmid is digested with *Sac*I and *Sal*I and the fragment (4931 bp) which contains the F gene-deficient region is subcloned into pUC18 to give pUC18/dFSS. This pUC18/dFSS is digested with DraIII and the digested fragment is recovered. The fragment is replaced with an F gene-containing DraIII fragment of pSeV18$^+$ to construct plasmid pSeV18$^+$/ΔF.

An exogenous gene is inserted, for example, into the *Nsi*I and *Ngo*MIV restriction enzyme sites in the F-deficient region of pUC18/dFSS. In order to clone exogenous genes into the region, for example, exogenous gene fragments can be amplified using an *Nsi*I-tailed primer and an *Ngo*MIV-tailed primer.

<2> Construction of helper cells inducibly expressing SeV-F protein

For the construction of a plasmid expressing the SeV-F gene, Cre/loxP induced expression plasmid, plasmid pCALNdLw is constructed through the amplification of the SeV-F gene by PCR and the insertion of the amplified product into the unique *Swa*I site of plasmid pCALNdlw (Arai, T. et al., J. Virol. 72: 1115-1121 (1998)), which is designed to express the gene product upon induction with Cre DNA recombinase.

A helper cell line, which expresses SeV-F protein, is established to recover infectious virus particles from the F gene-deficient genome. For example, LLC-MK2 cells, monkey kidney-derived cell line, which is often used for SeV propagation can be utilized. LLC-MK2 cells are cultured in MEM containing 10% heat inactivated fetal bovine serum (FBS), 50 U/ml Sodium Penicillin G and 50 μg/ml Streptomycin in an atmosphere containing 5% $CO_2$ at 37°C. Since SeV-F gene products have cytotoxicity, the plasmid, pCALNdLw/F, which is designed to induce the expression of the F gene product by *Cre* DNA recombinase, is transferred into LLC-MK2 cells using the Calcium Phosphate method with Mammalian Transfection Kit (Stratagene, La Jolla, CA) according to protocols known to those skilled in the art.

Ten μg of plasmid pCALNdLw/F is transferred into LLC-MK2 cells which are propagated to 40% confluence in a 10-cm dish and then the cells are cultured in 10ml MEM medium containing 10% FBS in an incubator with an atmosphere of 5% $CO_2$ at 37°C for 24 hours. Cells are scraped from the dish after 24 hours, suspended in 10 ml medium, and aliquoted to five 10-ml dishes so that, for example, 1 dish contains 5 ml, 2 dishes contain 2 ml, and 2 dishes contain 0.2 ml of cell suspension. Each cell is cultured in 10 ml MEM medium containing 1,200 μg/ml G418 (GIBCO-BRL) and 10% FBS for 14 days with a medium change every 2 days and stably-transfected cell lines are selected. G418 resistant cells, grown in the medium, are recovered using a cloning ring. Each recovered clone is propagated until it becomes confluent in a 10-cm dish.

The cells are cultured to confluence in 6-cm dishes and then infected with Adenovirus AxCANCre at m.o.i= 2 or 3 by the method by Saito et al. (Saito, et al., Nucl. Acids Res. 23: 3816-3821 (1995); Arai, T. et al., J. Virol. 72(2): 1115-1121 (1998)) to induce expression of F protein in each clone.

<3> Reconstitution and amplification of F gene-deficient SeV

The above-described plasmid introduced with the exogenous gene of pSeV18$^+$/ΔF is transfected into LLC-MK2 cells as follows. LLC-MK2 cells are seeded onto 100-mm petri dishes at a density of 5x $10^6$ cells/dish, incubated for 24 hours, and inoculated (m.o.i=2) (at m.o.i= 2 to 3, preferably 2) for 1 hour at room temperature with recombinant Vaccinia virus expressing T7 RNA polymerase (Fuerst, T.R. et al., Proc. Natl. Acad. Sci. USA 83: 8122-8126 (1986)) which has been treated with long wave UV (365 nm) and Solaren for 20 min. For UV exposure of the Vaccinia virus, for example, UV Stratalinker 2400 (Catalog No. 400676 (100 V), Stratagene, La Jolla, CA, USA) which is equipped with five 15-watt bulbs is used. Cells are washed three times and the plasmids pSeV18$^+$/ΔF-GFP, pGEM/NP, pGEM/P, and pGEM/L (Kato, A., et al., Genes Cells 1: 569-579 (1996)) are resuspended in OptiMEM (GIBCO) at ratios of 12 μg, 4 μg, 2 μg, and 4 μg/dish, respectively, and mixed with SuperFect transfection reagent (1 μg DNA / 5 μl of Superfect, QIAGEN). Mixtures are left standing at room temperature for 10 min and then added to 3 ml OptiMEM containing 3% FBS. The resulting mixture is added to the cells and incubated for 3 hours. The cells are then washed twice with serum-free MEM and incubated in MEM containing 40 μg/ml of cytosine β-D-Arabinofuranoside (AraC, Sigma) and 7.5 μg/ml trypsin (GIBCO) for 70 hours. These cells are harvested and the cell pellet is suspended in OptiMEM ($10^7$ cells/ml). After 3 cycles of freeze-thawing, the cells are mixed with lipofectin reagent DOSPER (Boehlinger Mannheim) ($10^6$ cells/25 μl DOSPER), and kept at room temperature for 15 min. LLC-MK2/F7 cells ($10^6$ cells/well, 12-well plate), one of the F expressive helper cells cloned above, are transfected with the mixture, cultured in serum free MEM (containing 40 μg/ml AraC and 7.5 μg/ml trypsin), and then the culture supernatant is collected.

In preparing deletion virus vectors, two different virus vectors having deletion of a different envelope gene in the viral genome of the vector may be transfected into the same cell. In this case, each deleted envelope protein is supplied through expression from the other vector, and this mutual complementation permits the generation of infective virus particles, which can replicate and propagate. Thus, two or more of the virus vectors of the present invention may be simultaneously inoculated in a combination that complement each other, thereby producing a mixture of each envelope deletion virus vector at a low cost and in a large scale. Because these viruses lacking an envelope gene have a smaller genome, they can maintain a long exogenous gene more stably. In addition, it is difficult for these viruses, which are intrinsically non-infective, to keep the status of co-infection after being diluted outside cells, and thus they are sterilized and less harmful to the environment.

Optionally, RNA-dependent RNA polymerase inhibitors may be administered after the administration of replicable Paramyxovirus vectors into individuals or cells to specifically arrest the propagation of the vectors without damaging the host. Such administration of RNA-dependent RNA polymerase inhibitors may become necessary upon termination of treatment and such.

The collected Paramyxovirus may be purified to be substantially pure. The purification can be carried out by known purification/separation methods including filtration, centrifugation, and column purification, or combinations thereof. The phrase "substantially pure" means that the virus is the major portion of a sample where it is present as a component. Typically, a sample can be confirmed to be a substantially pure virus vector when proteins derived from the virus vector occupies 10% or more, preferably 20% or more, more preferably 50% or more, more preferably 70% or more, more preferably 80% or more, and even more preferably 90% or more, of the total proteins (but excluding proteins added as carriers or stabilizers) in the sample. Specific examples of purification methods for Paramyxovirus include methods using cellulose sulfate ester or cross-linked polysaccharide sulfate ester (JP-B Sho 62-30752; JP-B Sho 62-33879; and JP-B Sho 62-30753), and those including adsorption of the virus with fucose sulfuric acid-containing polysaccharide and/or its degradation product (WO 97/32010).

The mammalian cell-infecting virus vectors encoding epitope-linked-β2m in an expressible manner express and secrete the epitope-linked-β2m in mammalian cells upon introduction of the vectors into the mammalian cells. The present invention provides a method for producing an epitope-linked-β2m, which comprises the steps of: (a) introducing a mammalian cell-infecting virus vector that encodes an epitope-linked-β2m in an expressible manner into mammalian cells, and (b) recovering the epitope-linked-β2m from the mammalian cells introduced with the vector or the culture supernatant thereof.

The epitope-linked-β2m expressed in mammalian cells forms a heterodimer with an endogenous MHC molecule or an MHC molecule exogenously expressed in the cells. The heterodimers are recovered to obtain a protein complex containing the epitope-linked-β2m. Specifically, this invention provides a method for producing an MHC class I/peptide complex containing the MHC class I heavy chain and epitope-linked-β2m, which comprises the steps of: (a) introducing a mammalian cell-infecting virus vector that encodes an epitope-linked-β2m in an expressible manner into mammalian cells, and (b) recovering the formed MHC class I/peptide complex from the mammalian cells introduced with the vector or from the culture supernatant thereof. More preferably, to prepare the MHC class I/peptide complex, the MHC class I heavy chain is overexpressed exogenously in mammalian cells, and the heavy chain is then made to form the complex with the epitope-linked-β2m. For example, a virus vector expressing the MHC class I heavy chain is introduced into the mammalian cells. There is no limitation on the virus vector, but preferably, Paramyxovirus vectors may be used. Alternatively, other virus vectors may be used. Moreover, mammalian cells wherein the vector is integrated into the chromosome, allowing constitutive or inducible expression of a desired MHC class I heavy chain, may be used. This enables intracellular overexpression of the desired MHC class I heavy chain to prepare the MHC class I/peptide complex. Alternatively, the MHC class I/peptide complex may be prepared through the binding of separately prepared epitope-linked-β2m and MHC class I heavy chain.

The MHC class I heavy chain may be expressed either as a membrane-bound form or a secretory form (or free form). Furthermore, the MHC class I heavy chain may be a wild-type protein or one with modified amino acid sequence. The secretory form MHC class I heavy chain may be prepared by expressing a protein with deleted membrane-binding region. Moreover, a desired peptide may be attached to the MHC class I heavy chain. For example, the addition of a biotinylation substrate peptide to the MHC class I heavy chain allows simple preparation of a tetramer of an obtained MHC class I/peptide complex using avidin via biotinylation of the expressed complex.

In the present invention, the phrase "biotinylation substrate peptide" refers to a peptide that serves as a substrate for biotinylation. There is no limitation on the biotinylation substrate. For example, any peptide may be used as the biotinylation substrate as long as an -NH$_2$ group present on an amino acid residue is arranged in a state to allow chemical reaction. For example, a saccharide portion may be chemically modified by Biotin hydrazide (Assay Design, Co., Ltd.), a lysine-NH$_2$ may be modified by Biotin HNS, an -SH group may be modified by Biotin BMCC, and a -COOH group may be modified by 5- (Biotinamido) -Pentylamine EZ-Link. A preferred biotinylation substrate peptide includes peptides that allow enzymatic biotinylation. For example, a peptide serving as a substrate for a biotinylation enzyme (biotin ligase) that specifically adds one biotin to an amino acid (for example, lysine) is preferred. Such enzyme includes, for example, Bir A, a biotin ligase derived from *E.coli*. Bir A recognizes a peptide sequence of 13 amino acids and adds one biotin to a lysine at the center of the peptide. Several kinds of sequences are known to serve as a substrate for Bir A, and include, for example, LGGIFEAMKMELRD (SEQ ID NO: 31) (Schatz, P., Biotechnology 11: 1138-1143 (1993); Crawford, F., Immunity 8: 675-682 (1998)).

Such an MHC class I/peptide tetramer can be constructed according to methods comprising the steps of: (a) biotinylating an MHC class I heavy chain that contains a biotinylation substrate peptide, and (b) assembling the biotinylated MHC class I heavy chain in the presence of avidin. Specifically, for example, a Bir A substrate peptide (BSP) sequence is attached to an MHC class I heavy chain, and then an MHC class I/peptide complex containing this MHC class I heavy chain is prepared. Then, the MHC class I heavy chain is biotinylated with Bir A enzyme. After purification of the biotinylated MHC class I/peptide complex by such as FPLC, it may be multimerized through reacting it at a 1:1 ratio with the biotinylating site of streptavidin. Streptavidin may be appropriately labeled with R-phycoerythrin (R-PE) , Cy 5, and such. The tetramerized MHC class I/peptide complex is extremely useful for the detection and quantification of CTL (epitope-specific CD8-positive T cells) due to its enhanced CTL-binding ability. Cells bound to the MHC classI/ peptide multimer can be detected by FACS and such.

Thus, the present invention relates to a method for detecting and quantifying antigen-specific T lymphocytes utilizing a vector of the present invention, or epitope-linked-β2m or MHC class I/peptide complex expressed by the vector of the present invention. Furthermore, the present invention provides a detection agent for antigen-specific T lymphocytes comprising a vector of the present invention, or epitope-linked-β2m or MHC class I/peptide complex produced by using the vector. Moreover, the present invention provides a use of the vectors of the present invention, or epitope-linked-β2m or MHC class I/peptide complex produced by using the vectors, for the detection of antigen-specific T lymphocytes.

For example, the MHC class I/peptide tetramer can be used for a simple and highly sensitive quantification of epitope-specific CD8-positive T cells. This method for detecting antigen-specific T lymphocytes using the tetramer comprises the steps of: (1) presenting the tetramer in a sample containing T lymphocytes, and (2) detecting cells bound to the tetramer. It is possible to quantify the antigen-specific T lymphocytes by quantifying the cells bound to the tetramer.

To quantify epitope-specific CD8-positive T lymphocytes in individuals, for example, when the epitope is derived from HIV, a tetramer that displays the HIV epitope is added at an amount of 20 μg/ml as the MHC class I/peptide complex to 1x 10$^6$ peripheral blood mononuclear cells (PBMC) from a HIV-infected patient, and incubated at 37°C for 15 min and then at 4°C for 20 min. Herein, the optimal quantity of the tetramer and the optimal temperature vary depending on each tetramer, and thus are appropriately adjusted. Then, the cells are double-stained with anti-CD8-APC and analyzed by flow cytometry to determine the frequency of tetramer-positive cells among total CD8-positive T cells. This method may be applied to cells isolated from various tissues such as lymph node, in addition to PBMC.

The cells stained with the tetramer according to the above procedure may be further stained with anti-PE-magnetic beads (Daiichi Pure Chemicals) to isolate epitope-specific cells using a magnetic cell sorter (Daiichi Pure Chemicals). Thus, the tetramer of the present invention is also useful for the isolation of epitope-specific cells. Specifically, antigen-specific T lymphocytes can be isolated through the steps of: (1) presenting a tetramer of the present invention in a sample containing T lymphocytes, and (2) separating cells bound to the tetramer.

Moreover, a vector of the present invention or epitope-linked-β2m expressed by the vector may be used for *in vitro* CTL assay. Target cells for the CTL assay generally include cell lines derived from the same individual as the CTL. For example, cells of Epstein-Barr virus-transformed B cell line (auto-lymphoblastoid cell line, auto-LCL) pulsed with a synthetic peptide may be used as the target cells. Alternatively, instead of using the synthetic peptide, epitope-linked-β2m prepared by the vector of the present invention may be added or the virus vector expressing the epitope-linked-β2m may be infected into the auto-LCL. In fact, such auto-LCL was confirmed to have a cytotoxic activity. Cells of human cell lines can be used similarly through co-infection of virus vectors that express a desired MHC class I heavy chain (membrane-bound form) and an epitope-linked-β2m, which will be displayed on the MHC class I heavy chain, respectively.

Moreover, epitope-linked-β2m may be used to establish epitope-specific cytotoxic T lymphocytes (CTL). Such epitope-specific CTL can be established via specific single or multiple stimulations *in vitro.* For example, HIV Nef138-10-specific CTL can be established by arresting the growth of HIV-infected individual's peripheral blood mononuclear cells (PBMC) by irradiation, stimulating the PBMC with stimulator cells (derived from the same individual) pulsed with the HIV Nef138-10 peptide, and then co-culturing the cells in the presence of IL-2. Herein, culture supernatant of cells infected with a virus vector that expresses the epitope-linked-β2m of the present invention (i.e., free form epitope-linked-β2m protein) was demonstrated to be effective as synthetic peptides. Epitope-specific CTL may also be established by infection of a virus vector expressing the epitope-linked-β2m to the stimulator cells.

Furthermore, the epitope-linked-β2m and MHC class I/peptide complexes (monomers or multimers) of the present invention can be used for a protein chip by binding them to a supporting-base of protein chips, for examfple, via a method other than utilizing biotin-avidin binding.

Human MHC class I molecules are preferably used as the MHC class I heavy chain when it is applied to humans , but not limited thereto. Human MHC class I molecules are called human leukocyte antigens (HLA) and are quite rich in variety. Any of these molecules can be used as the HLA in the present invention. For example, the present invention may be applied to any type of HLA whose desired epitope has been identified in infectious diseases and cancers, and they may be used as order-made or tailor-made reagents.

In particular, HLA-A24 (approximately 60 to 70%), HLA-A2 (approximately 40%), and HLA-A26 (approximately 20%) are high frequent HLA types in Japanese, followed by HLA-A11 (20%), HLA-A31 (up to 20%), and HLA-A33 (up to 20%). These HLA types are particularly preferred when applied to Japanese.

For the purpose of developing reagents or medicaments applicable to a certain extent of many unspecified individuals in, for example Japanese, HLA types having 5% or more allele frequency among Japanese population (namely, 10% or more HLA type frequency among Japanese) is preferred. Such HLA types include:

| A locus | B locus |
|---------|---------|
| A*0201 | B*0702 |
| A*0206 | B*1501 |
| A*1101 | B*3501 |
| A*2601 | B*4001 |
| A*31012 | B*4002 |
| A*3303 | B*44031 |
| | B*4601 |
| | B*52011 |
| | B*5401 |

In the Examples, the present inventors used A24-restricted HLA class I Heavy chain as the MHC class I molecule and an HIV-1 derived epitope displayed on the A24-restricted HLA class I molecule as the epitope. Approximately 60 to 70% of the Japanese population have the genotype HLA-A*2402. Therefore, the A24-restricted HLA class I heavy chain, in particular, A*2402 (Litte, A.-M., Immunogenetics 35: 41-45 (1992)) is suitable in the present invention.

The epitope-linked-β2m and MHC class I/peptide complexes obtained according to the present invention may be used to induce antigen-specific cellular immune response. The present invention provides an agent to induce antigen-specific cellular immune response that comprises epitope-linked-β2m or MHC class I/peptide complex obtained according to the present invention. Furthermore, the present invention relates to the use of the epitope-linked-β2m or the MHC class I/peptide complexes obtained according to the present invention to induce antigen-specific cellular immune response.

The inventors of the present invention constructed an epitope-linked-β2m derived from the HIV-1 Nef protein presented by HLA-A*2402 as an example using the SeV expression system, and verified that the epitope-linked-β2m binds as a secretory protein to the HLA-A*2402 molecules on the cell surface, resulting in an efficient antigen presentation to CTL. The epitope-linked-β2m produced by the virus vectors of the present invention was suggested to be useful as a protein preparation. After purification and removal of virus contaminants from the epitope-linked-β2m, a clinically useful inducer of antigen-specific cellular immune response can be obtained. The present invention provides a pharmaceutical composition comprising the epitope-linked-β2m or MHC class I/peptide complexes produced by the virus vectors of the present invention.

The method for inducing antigen-specific cellular immune response using epitope-linked-β2m or MHC class I/peptide complexes provided by the present invention specifically includes the step of contacting the epitope-linked-β2m or MHC class I/peptide complexes of the present invention with (epitope-specific) CD8-positive T cells.

For example, epitope-linked-β2m of the present invention is useful for establishing *in vitro* epitope-specific CTL cell lines. Epitope-specific CTL cell lines can be established by one to several rounds of specific stimulations *in vitro*. For example, to establish HIV Nef138-10-specific CTL cell lines, PBMC from HIV-infected individuals are irradiated to arrest their proliferation, stimulated with stimulator cells (derived from the same individual) pulsed with the HIV Nef138-10 peptide, and then co-cultured in the presence of IL-2. Herein, synthetic peptides are generally used for the stimulation; however, a similar effect can be achieved by culture supernatants of cells infected with the epitope-linked-β2m.

In addition, epitope-linked-β2m of the present invention is useful to pulse target cells in CTL assays *in vitro*. Target cells in the CTL assays may be pulsed with culture supernatants of cells infected with the epitope-linked β2m, which gives the same result as with the synthetic peptide. Generally, a complex of an MHC class I heavy chain (membrane-bound form) + β2m + peptide is already expressed on the cell surface. Thus, the pulsing with the synthetic peptide is intended to exchange the synthetic peptide with the peptide portion of the complex. On the other hand,' exchange of the β2m and peptide portion of the complex is suggested to occur when pulsed with the epitope-linked-β2m.

Epitope-linked-β2m of the present invention is also useful to pulse patient's dendritic cells *ex vivo*. Peripheral blood mononuclear cells are isolated from a patient and then differentiation to dendritic cells is induced by a standard method. These dendritic cells are pulsed with the epitope-linked-β2m to subcutaneously administer them to the patient. As a result, the epitope-linked-β2m is expected to induce epitope-specific CTL due to its expression on the cell surface as a complex with an MHC class I heavy chain (membrane-bound form) similarly as in the *in vivo* experiment. Since the dendritic cells pulsed with the epitope-linked-β2m is expected to express the epitope on the MHC class I heavy chain (membrane-bound form) for a longer time than cells pulsed with a peptide, it may be used as a "cellular vaccine (therapeutic vaccine)". Moreover, epitope-specific CTL may be induced by direct administration of the epitope-linked-β2m of the present invention as a protein preparation.

Furthermore, mammalian cell-infecting virus vectors that encode epitope-linked-β2m in an expressible manner themselves may be used to induce antigen-specific cellular immune response. The present invention relates to inducers of antigen-specific cellular immune response comprising the mammalian cell-infecting virus vector that encodes the epitope-linked-β2m in an expressible manner. Moreover, the present invention relates to the use of the mammalian cell-inducing virus vector that encode the epitope-linked-β2m in an expressible manner for the induction of antigen-specific cellular immune response. The virus vectors of the present invention are useful for gene therapy. The present invention provides pharmaceutical compositions comprising the mammalian cell-infecting virus vector that encodes the epitope-linked-β2m in an expressible manner. The virus vector is useful to induce antigen-specific immune response for infectious diseases, cancers, and such, due to its antigen-specific cellular immune response inducing ability. Applications of the virus vectors of the present invention to gene therapy may be achieved through gene expression by either a direct (*in vivo*) administration or an indirect (*ex vivo*) administration of the virus vectors.

For example, in the process of *in vitro* establishment of epitope-specific CTL cell lines, stimulation of stimulator cells may be achieved by infecting them with a virus vector that expresses epitope-linked-β2m instead of using a synthetic peptide.

The virus vectors of the present invention may also be used to pulse target cells in an *in vitro* CTL assay. Auto-LCL cells infected with a virus vector expressing epitope-linked-β2m instead of a synthetic peptide may be used as target cells in the CTL assay. In fact, the cells were confirmed to have cytotoxic activity in the CTL assay. In addition, to use human cell lines as target cells, they may be co-infected with a virus vector expressing a desired MHC class I heavy chain (membrane-bound form) and a virus vector that expresses an epitope-linked-β2m presented by the MHC class I heavy chain.

Furthermore, the vectors of the present invention may be used for *ex vivo* gene transfer into patient's dendritic cells. Peripheral blood mononuclear cells (PBMC) are isolated from a patient and induced to differentiate into dendritic cells by a standard method. The dendritic cells are infected with a virus vector expressing epitope-linked-β2m, and then inoculated to the patient subcutaneously. The important point here is how long the expression of a pulsed peptide or the epitope-linked-β2m protein continues on the surface of the pulsed dendritic cells. In fact, when the cells are pulsed with a synthetic peptide, the expression of the epitope on the cell surface of the dendritic cells disappears in a short term. In contrast, a long time expression of the epitope-linked-β2m can be achieved via the infection of the dendritic cells with the virus vector to allow intracellular expression of the epitope-linked-β2m. Furthermore, the vectors of the present invention are also useful for *in vivo* gene therapy wherein epitope-specific CTL is induced by *in vivo* administration of the vectors.

Target cells for gene transfer using the vectors preferably include those having antigen presenting ability. Such cells particularly include dendritic cells (DC). For example, a desired MHC class I/peptide complex may be formed on dendritic cells by introducing a vector of the present invention into the dendritic cells *ex vivo*. The resulting cells may be used for the activation of antigen-specific T cells. The present invention relates to mammalian cells infected with a mammalian cell-infecting virus vector encoding epitope-linked-β2m of the present invention in an expressible manner. Desired isolated cells having antigen-presenting ability may be used as the mammalian cells. In particular, a large amount of MHC class I/peptide complexes may be formed by introducing an exogenous gene encoding an MHC class I heavy chain into the cells. The gene that encodes the MHC class I heavy chain may be either encoded by an expression vector outside the chromosome or integrated into cellular chromosome. A secretory form MHC class I/peptide complex can be produced in the cells by intracellularly expressing a secretory form MHC class I heavy chain. Such cells may be particularly useful for the production of the secretory form MHC class I/peptide complex. When a membrane-bound form MHC class I heavy chain is intracellularly expressed, a membrane-bound MHC class I/peptide complex is expressed on the cell surface. Such cells have an excellent antigen-presenting ability to epitope-specific CTL. Furthermore, the present invention relates to cells presenting epitope-linked-β2m produced by the method of the present invention on the cell surface. Such cells can be prepared, for example, by adding the epitope-linked-β2m produced by the method of the present invention to the cells. For example, dendritic cells may be prepared from a patient and induced *in vitro* to differentiated dendritic cells according to methods known to those skilled in the art. The resultant cells are mixed with the epitope-linked-β2m produced by the method of the present invention. This results in the formation of a complex of the epitope-linked-β2m and MHC class I heavy chain on the cell surface of the dendritic cells derived from the patient. These cells also have the ability to present antigens to epitope-specific CTL. The produced cells presenting the desired epitope may be put back into the patient's body to induce epitope-specific immune response. Namely, these cells can be used as vaccines. Thus, the epitope-linked-β2m produced according to the method of the present invention is useful as a pharmaceutical to induce antigen-specific immune response in patients. To produce cells that present epitope-linked-β2m on the cell surface, the cells are pulsed with the epitope-linked-β2m produced by the present method. Before the pulsing, pre-existing epitopes on the cells can be removed by acid treatment of the cells. The acid treatment can be carried out through exposing the cells in an acidic environment so that the epitope peptides presented on the cell surface are significantly dissociated. The acid treatment may be carried out, for example, by treating the cells with a peptide-stripping buffer (0.13 M citric acid (pH 3), 66 mM $Na_2HPO_4$, 150 mM NaCl, 17 mg/ml Phenol Red) at 4°C for 1 min, followed by neutralization with enough culture medium (for example, RPMI) . Those skilled in the art are able to perform the acid treatment via other protocols achieving similar effect. The present invention provides pharmaceutical compositions comprising the above-described cell infected with the mammalian cell-infecting virus vector encoding epitope-linked-β2m in an expressible, or the cell presenting the epitope-linked-β2m produced according to the method of the present invention.

In principle, since most of the somatic cells endogenously express the MHC class I, there is no need to exogenously express an MHC class I heavy chain in the production of above cells, but only express or add epitope-linked-β2m. A tailor-made medicine is realized through MHC typing of the patient and expression or addition of epitope-linked-β2m selected in accordance with the MHC type. For example, when the patient has the A24-restricted MHC type, then the use of β2m fused with an epitope presented by an A24-restricted T cell is expected to produce a more significant effect in the therapy. It is preferred that the MHC type is identical in serotype or in genotype, and more preferably in genotype. Since approximately 70% of Japanese have the HLA-A*2402 genotype, it is possible to produce a medicine particularly suited for the application to Japanese through identifying an epitope presented by cells having HLA-A*2402 and designing an epitope-linked-β2m using the identified epitope. Furthermore, the most adapted therapeutic strategy for each patient can be determined through the analysis of epitopes corresponding to many kinds of MHC types.

The mammalian cell-infecting virus vectors, epitope-linked-β2m and MHC class I/peptide complexes obtained by the virus vectors, cells introduced with the vector, or cells having epitope-linked-β2m on the cell surface of the present invention may be prepared as a composition in combination with, if necessary, desired pharmaceutically acceptable carriers or vehicles. The phrase "pharmaceutically acceptable carriers or vehicles" refers to materials capable of being administered with the vectors, epitope-linked-β2m or MHC class I/peptide complexes, or the above-described cells of the present invention, and that do not significantly interfer their activities. For example, a composition can be made by proper dilution of a mammalian cell-infecting virus vector of the present invention, or an epitope-linked-β2m or MHC class I/peptide complex obtained by the vector with physiological saline or phosphate-buffered saline (PBS). When a Paramyxovirus vector has been propagated in chicken eggs, the composition may contain allantoic fluid. Moreover, the above cells may be suspended in physiological saline, PBS, or culture solution. In addition, the compositions of the present invention may contain carriers or vehicles such as deionized water and 5% dextrose solution. Furthermore, it may contain other materials including vegetable oils, suspending agents, detergents, stabilizers, and sterilizers. Moreover, preservatives and other additives may be used for the compositions. The compositions of the present invention are useful as reagents and medicines. The compositions are further useful as vaccines. The present invention also relates to the use of the vectors of the present invention, epitope-linked-β2m or MHC class I/peptide complexes obtained by the vectors, or the compositions comprising the above cells as reagents, medicines, or vaccines. To enhance immunogenicity, the vaccine compositions may contain immune accelerators including cytokines, cholera toxins, and salmonella toxins. Furthermore, the vaccines may be combined with adjuvants including alum, incomplete Freund's adjuvant, MF59 (oil emulsion), MTP-PE (muramyl tripeptide derived from Mycobacterium cell wall), and QS-21 (derived from soapbark tree *Quilaja saponaria*).

Moreover, cytokines can be effectively combined to enhance the adjuvant effect of the pharmaceutical compositions of the present invention for administration. Such combinations include: i) combination of IL-2 and single-stranded IL-12 (Proc. Natl. Acad. Sci. USA 96(15): 8591-8596 (1999)), ii) combination of IL-2 and interferon-γ (US Pat. No. 5,798,100), iii) GM-CSF alone, and iv) combination of GM-CSF and IL-4 for the treatment of brain tumors (J. Neurosurgery 90(6): 1115-1124 (1999)).

The vaccines of the present invention may be applied, for example, to tumors, infectious diseases, and other general diseases. For the treatment of infectious diseases, for example, epitopes of antigen proteins of infectious microorganism are analyzed, and then β2m fused with the epitopes are produced. Antigen proteins include, for example, envelope of virulent strain type H5N1 for influenza; for Japanese encephalitis, the envelope protein (Vaccine 17 (15-16) : 1869-1882 (1999)); HIV gag or SIV gag proteins (J. Immunology 164: 4968-4978 (2000)), HIV envelope proteins, the Nef protein and other virus proteins for AIDS; B subunit (CTB) of cholera toxin (Arakawa, T. et al. , Nature Biotechnology, 16(10): 934-8 (1998); Arakawa, T. et al., Nature Biotechnology, 16(3): 292-7 (1998) ) as an example of cholera antigens; glycoprotein of rabies virus (Lodmell, D.L. et al. , Nature Médicine 4 (8) : 949-52 (1998) ) as an example of rabies antigens; and capsid protein L1 of human papilloma virus type 6 (J. Med. Virol 60: 200-204 (2000)) as an example of cervical cancer antigens. Furthermore, the present invention may be applied to pathogenic Paramyxovirus such as Measles virus and epidemic parotiditis virus that highly require the use of vaccines. Moreover, epitopes of antigen proteins including JE-E antigen protein of Japanese encephalitis (JP-A Sho 64-74948, JP-A Hei 1-285498), gD2 protein of human herpes simplex virus (JP-A Hei 5-252965), hepatitis C virus-derived peptide (JP-A Hei 5-192160), and pseudorabies virus-derived polypeptide (Published Japanese Translation of International Publication No. 7-502173), may also be used. For example, cells from patients infected with these pathogenic microorganisms are analyzed to identify epitopes of the antigen proteins presented on antigen-presenting cells (APC). An epitope for the desired HLA type can be identified by selecting a proper HLA type.

It is clinically significant in treating tumor to develop diverse and new therapeutic strategies and methods, focusing on tumor-specific antigens. For example, in tumor therapy, anepitope-linked-β2m may be expressed in antigen-presenting cells (APC) , such as tumor cells or DC cells, using a vector of the present invention, or protein products, such as epitope-linked-β2m and MHC class I/peptide complex, may be administered.

General means to develop a vaccine therapy includes the use of DC cells known to have antigen-presenting ability to helper T cells (Th) as well as high antigen-presenting ability to CTL via MHC class I molecules (Mayordomo, J.I. et al., Nature Med. 1(12): 1279-1302 (1995) ) . As an example of tumor immunotherapy, studies to establish a tumor vaccine therapy utilizing the MAGE antigen, which expression is confirmed in a variety of malignant tumors, as a target are in progress by Yasushi Fuji et al. (National Hospital Kyushu Cancer Center). The method was applied to 7 cases including 5 cases of recurrent gastric tumor and a case of recurrent esophagus tumor. As a result, shrink of metastatic lymph node, decrease in tumor markers, and improvement of clinical symptoms (hoaseness) were observed in the recurrent esophagus tumor case. In the gastric tumor cases, decrease in tumor markers in 4 of 6 cases was observed. No side effect was observed, suggesting safety of the tumor immunotherapy. Moreover, in many cases, improvement of clinical symptoms and decrease in tumor markers were observed, indicating the possibility that it serves as an effective therapeutic method through selection of the administration route, adaptable cases, and such. Clinical studies on the DC vaccine therapy using the MAGE-3 peptide have been practically started, and are suggested to be a safe and tumor-specific immunotherapy against progressive recurrent digestive organ cancer cases. However, for preventive administration of such vaccines to many patients, it is clearly troublesome and unpractical to find an optimal condition for administration of the dendritic cells as "natural adjuvant" to each patient. Therefore, efficient methods are desired in the art (Yamagishi, H. et al., Oral presentation in the General meeting of Japan society of Cancer Therapy: October 12 (1999), Gifu City) . The application of the present invention to such a tumor vaccine therapy may be quite effective.

The prevention of tumorgenesis triggered by viruses may be achieved by preventing infection using a virus vaccine. Thus, virus-caused tumors can be more practically prevented compared with those caused due to other factors. For example, hepatitis C virus (HCV) associated with hepatic tumors, human papillomavirus (HPV) associated with uterine cancer, and HTLV-1 associated with adult T cell leukemia are candidates for vaccines aiming at prevention and therapy of infections. Hepatic tumors share a high ratio in Japanese tumors : Infection by hepatitis C virus occurs non-orally, and at high frequency gets chronic even in healthy adults with normal immunocompitence. Approximately 20% of the infected individuals are predicted to suffer chronic hepatitis or cirrhosis. Moreover, many of the cirrhosis patients acquire liver cell cancer. While interferon treatment of hepatitis C made it possible to cure a part of the patients, its efficacy was not as initially expected and even now, there is no effective therapy for more than half of the patients. Generally, viral infections are prevented by inducing neutralizing antibodies via vaccination. However, hepatitis C virus is susceptible to mutation and the existence of a neutralizing antibody that interrupts its infection has not been proven so far. Virus infections are known to be protected by inducing CTL in addition to the induction of neutralizing antibodies. The administration of the vectors of the present invention is expected to induce activation of CTL in such virus infections.

Moriyama, T. et al. (Jichi Medical School) have been studying methods using genes themselves that encode pathogenic antigens as vaccines (DNA vaccines) as a novel method to induce CTL. The method has problems such as insufficient expression and the administration site being limited to the muscles as described in their articles (Kurane, I. "DNA vaccine, present situation and new findings", Current Concepts in Infectious Diseases 19 (3) : 6-9 (2000)). Another article describes a cancer vaccine utilizing HER2, a membrane type glycoprotein having tyrosine kinase activity, that is found overexpressed in breast cancers (Kageyama, S., Watanabe, M., Hiasa, A. , and Suku, K. , "Cancer and immunity, cancer-specific immune therapy, vaccine therapy against breast cancer using a HER-derived peptide", New Horizon for Medicine 32 (5) : 1167-1172 (2000)). The virus vectors. provided by the present invention is expected to exert higher effect than these DNA vaccines. Highly efficient vaccines may be provided according to the present invention by constructing vectors using virus vectors that have a wide expression range among mammal tissues (adenovirus vectors, adeno-associated virus vectors, herpes simplex virus vectors, retrovirus vectors, Lentivirus vectors, Semliki forest virus vectors, Sindbis virus vectors, vaccinia virus vectors, Sendai virus vectors, etc.).

Although uterus cancer is a female-specific tumor, the significance to develop vaccines for the prevention and therapy of HPV infection is the same as other tumors. The main infection route of HTLV-1 is mother-to-child transmission. However, there are also other routes. Although the pathogenicity of recently discovered HGV is unclear, it is widespread in society like HCV, indicating the need to prevent such virus at the perspective of public health. Thus, such virus is also a target for the application of the present invention.

Investigations concerning applications of antigen-presenting cells and the route of administration are also important. The most extensively studied route of administration for vaccine therapy comprises the steps of: (1) isolating the small amount of DC cells contained in the peripheral blood of a patient; (2) culturing and amplifying them in vitro; and (3) returning them into the patient's body via intravenous injection. This method utilizes the ability of DC cells to present antigens via MHC class I molecules to CTL. This system requires respectable facilities and time for culture. Recently, vaccine therapy utilizes skin is featured. The investigations of anti-virus vaccine therapy and cancer vaccine therapy utilizing dendritic cells including Langerhans cells (LC) that were recently found to efficiently present endogenous antigens, such as virus antigens and cancer antigens, to CTL via MHC class I molecules on the cell surface are attracting attention. Since many LC cells are on the epidermis of skin, efficient DNA vaccine therapy may be developed by applying virus peptides or genes encoding antigens on the skin. However, LC cells in normal skin are in the dormant state, and have low antigen presenting activity to Th and CTL, as well as poor mobility into the lymph node. Therefore, it is difficult to realize a virus vaccine therapy or cancer vaccine therapy using normal skin. To solve these problems, Naohiro Seo and Masahiro Takigawa (Hamamatsu Univ. School of Medicine) along with colleagues demonstrated that LC cells on the skin become activated, then move into the lymph node and efficiently present antigens to Th cells by disrupting the most external skin layer, the corneum barrier, through 8 to 15 times repeated tape stripping (TS) (Japanese Patent Application No. Hei 10-316585, "Percutaneous peptide immunization via corneum barrier-disrupted murine skin for experimental tumor immunoprophylaxis"; Proc. Natl. Acad. Sci. USA 97: 371-376 (2000)). This method expands the possibility of virus vaccines and cancer therapy due to its potential to enable application of virus peptides, cancer peptides or antigen DNAs to the barrier-disrupted skin. These administration methods can be applied to the present invention.

To accomplish more confident clinical applications, it is important to construct human HLA-restricted cancer-specific CTL cell lines, and clone genes encoding cancer-reactive CTL-inducing antigens recognized by the cell lines to develop target molecules for clinically applicable tumor-specific immunotherapy against cancer patients. Immune response is expected to be induced more efficiently by identifying an epitope presented by an HLA of the same type as that of a patient, and producing a vector or peptide vaccine of the present invention using the identified epitope.

Cancers that frequently occur in Japanese include, for example, lung cancer, digestive tract cancer (esophagus, stomach, and colon cancers), liver cancer, head and neck cancer, breast cancer, uterine cancer, oophoroma, nephoroma, and leukemia. Among'these cancers, it is clinically useful to choose the tissue type of squamous cell carcinoma and adenocarcinoma as therapeutic targets, because they share most of these cancers in Japanese. Epidermal cancers share not only the majority of adult malignant tumors in Japanese, but also occur most frequently in the world. Therefore, epitopes of epidermal cancer cell-specific antigens are suitably used in the present invention. Moreover, as for HLA, the identification of HLA-A24 (approximately 60% of cancer patients) , HLA-A2 (approximately 40%), and HLA-A26 (approximately 20%)-restricted CTL-recognized cancer-regressive antigens are particularly important, since these HLA types are also dominant in Japan. Following these types, identification of antigens intended to HLA-A11 (20%), HLA-A31 (up to 20%), and HLA-33 (up to 20%) are also important. Ninety five percent or more Japanese possess at least any one of HLA-A24, -A2, -A26, -A31, and -A33. Furthermore, these HLA alleles are distributed widely beyond the ethnic difference. Therefore, it is preferred to identify genes encoding cancer-reactive CTL-inducing antigens from cells having these HLA types, and apply them to the present invention.

Kyogo Ito (Medical school of Kurume Univ.) et al. have established many specific killer T cells against human HLA class-restricted epidermal cancers (adenocarcinoma and squamous cell carcinoma) which frequently occur among Japanese, and cloned genes encoding antigens recognized by the cells and that have the ability to induce epidermal cancer-reactive CTL. Furthermore, they identified cancer antigen peptides encoded by the genes, and have been analyzing their *in vitro* killer T cell-inducing abilities (Tou, U., Yamana, H., Sueyoshi, S., Shintani, F., Tanaka, T., Kubota, M., Mine, T.', Sasahara, H., and Ito, K., "Cloning and analysis of an antigen gene from the peripheral blood lymphocytes after the specific adoptive immunotherapy by a local injection of CTL from a patient carrying esophagus cancer", The Japanese Society of Gastroenterological Surgery 33 (7) : 1191 (2000)). Hitherto, 4 genes (SART-1 to SART-4) and 3 genes from squamous cell carcinoma and adenocarcinoma cDNA libraries, respectively, were cloned and their coding proteins were analyzed. Selective apoptosis was induced in cancer cells introduced with the SART-1 gene. In addition, the HLA-A24-restricted peptide (SART-1 690-698) strongly induced CTL, and the HLA-A26-restricted peptide (SART-1 736-744) induced CTL in cancer patients with HLA-A2601, -A2602, or -A2603 (Yamana, H. and Ito, K. (Medical School of Kurume Univ.), "Antigen peptide therapy of tumors; cancer immunotherapy using the SART-1 antigen peptide", Journal of Clinical and Experimental Medicine 190 (2) : 129-133 (1999); Inoue, K. (Research Institute of National Cancer Center) , Nakao, M. , Matsunaga, K., Matsuoka-kikuchi, S., Yamana, H. , and Ito, K. (Medical School of Kurume Univ.), "Induction of CTL in peripheral blood lymphocytes from HLA-A26-positive healthy subjects and cancer patients with different HLA subtypes, using the SART-1 peptide", General meeting abstracts of Japanese Cancer Association). Furthermore, the 140 kD SART-3-reactive cancer-reactive CTL-inducing antigen was found to selectively express in proliferating cells and also in the nucleus of cancer cells, and two nonapeptides that have CTL-inducing ability to the lymphocytes of HLA-A24-positive cancer patients were identified within the antigen (Yamana, H., Sasatomi, T., Miyagi, Y., Tou, U., Shiromizu, K., and Ito, K. (Medical School of Kurume Univ.), Japan Surgical Society (supplement) 101: 417 (2000)). In the interest of the expression of these cancer-reactive CTL-inducing antigens at the protein level in various cancers, the SART-1 antigen was expressed in 60 to 80% of squamous cell carcinomas and in 40 to 50% of adenocarcinomas except for breast cancer; SART-2 in more than 60% of squamous cell carcinomas; and SART-3 in most malignant tumors including adenocarcinomas and squamous cell carcinomas. In contrast, these antigens are not expressed in normal tissues except the testicle (Ito, K., Shichijo, S., and Yamana, H. (Medical School of Kurume Univ.), "Tumor immunity 9, Human cancer-specific T cell-recognized antigen 2, Squamous cell carcinoma-reactive CTL-inducing antigen SART-1 and peptide vaccines", Immunology Frontier 9 (3) : 195-204 (1999)). HLA-A26 and HLA-A*2402 are shared in 22% and approximately 60%, respectively, in Japanese. Therefore, these peptide vaccines are expected to be applicable to many squamous cell carcinoma patients in Japan. Phase 1 clinical studies using the above peptides are being planned in Kurume Univ. (Yamana, H. and Ito, K. (Medical School of Kurume Univ.), "An explanation of guideline to clinical studies", A proposal regarding the first clinical studies on tumor peptide vaccines, Cellular Molecular Medicine 1(1): 89-95 (2000)). The application of the present invention may be useful for epitopes derived from these antigen peptides.

An effective immunotherapy can be conducted by applying genes encoding the above-described peptide antigens to the present invention. Apart from these antigens, epitopes include the cancer antigen Muc-1 and the Muc-1-like mucin tandem repeat peptide (US Pat. No. 5, 744, 144), and the melanoma gp100 antigen. Immunotherapy using these genes has been applied to a variety of cancers including breast cancers, colorectal cancers, pancreatic cancers, prostate cancers, lung cancer, etc. In addition, the gene encoding the above-mentioned tumor antigen peptide HER2 was found to be overexpressed or amplified in about 20 to 40% of cases of breast cancers, oophoromas, stomach cancers, and non-small cell pulmonary carcinomas, indicating high tendency of tumor specificity. Two nonapeptides (HER2p63-71 and HER2p780-788) originating from HER 2, which peptides are derived from dendritic cells purified from peripheral blood mononuclear cells of oophoroma patients and normal healthy subjects are exemplified as epitopes of the present invention (Eur. J. Immunol. 30: 3338-3346 (2000)). Moreover, the vectors or peptides of the present invention may be applied to cancer vaccine therapy for CEA-positive progressive solid tumors using CEA epitope peptides (Kim, C. et al., Cancer Immunol. Immunother. 47: W 90-96 (1998)). For example, large amounts of peripheral blood mononuclear cells may be isolated from a patient by selected collection of blood components, dendritic cells are induced by the addition of IL-4 and GM-CSF from the mononuclear cell fraction, induced dendritic cells are then introduced with CEA epitope peptide produced by the vector of the present invention or the vector itself, and finally the dendritic cells are subcutaneously administered as "DC vaccine" (Okamoto, K., Shirokazu, T., Sakakura, C., Otsuji, E.,Kitamura, K., and Yamagishi, K. (Kyoto Prefectural University of Medicine), "A case of bone metastatic lung cancer wherein dissociation between serum CEA values and anti-tumor effect was achieved via CEA-specific active immunotherapy", International Association of Surgeons and Gastoenterologists).

Optionally, the present invention may be applied to general diseases. In the interest of diabetes, for example, an insulin fragment peptide may be used as an epitope to type I diabetes animal model (Coon, B. et al., J. Clin. Invest, 104(2): 189-194 (1999)).

Compositions comprising a vector of the present invention, or an epitope-linked-β2m or MHC class I/ peptide complex obtained by the vector, may be administered at a quantity sufficient to at least partially induce antigen-specific cellular immune response. However, the administration dose of the protein or expression level of the gene should be determined by considering its effective level and intoxication level. The route of administration may be appropriately selected from, for example, percutaneous, pernasal, perbronchial, intramuscular, intraperitoneal, intravenous, intraarticular, and subcutaneous administrations, but is not limited thereto. They may be administered local or systemically. The induction of cellular immunity can be detected by such as CTL assay as described in the present invention.

The expression levels of genes introduced into cells using a vector of the present invention may be assayed by methods known to those skilled in the art. Transcription products from the genes may be detected and/or quantified by, for example, Northern hybridization, RT-PCR, or RNA protection assay. The detection by Northern hybridization and RNA protection assay can be also performed *in situ*. Western blotting, immunoprecipitation, RIA, ELISA, Pull-down assay, and such, using antibodies may be conducted for the detection of translation products. Moreover, for easier detection of expression products', tags may be attached to proteins to be expressed or reporter genes may be inserted so that they are expressed. Examples of the reporter genes include β-galactosidase, CAT, alkaline phosphatase, and GFP proteins; however, they are not limited thereto.

The dose of the epitope-linked-β2m or MHC class I/peptide complexes may vary depending on the disease, body weight, age, sex, and symptom of the patient, the purpose of administration, the type of the administered composition, the route of administration, and such, but it can be appropriately determined by those skilled in the art. The dose may be, for example, within the range of 10 ng/kg to 100μg/kg, preferably 100 ng/kg to 50 μg/kg, and more preferably 1 μg/kg to 5 μg/kg. When a combination of multiple epitopes are used for administration, each of the epitopes may be administered at the above dose. The Epitope-linked-β2m or the MHC class I/peptide complexes may be properly combined with pharmaceutically acceptable carriers.

The dose of the vectors may vary depending on the disease, body weight, age, sex, symptom, purpose of administration, transgene, and such, but it can be appropriately determined by those skilled in the art. A Paramyxovirus vector is preferably administered at a titer within the range of about $10^5$ pfu/ml to about $10^{11}$ pfu/ml, more preferably about $10^7$ pfu/ml to about $10^9$ pfu/ml, and most preferably about 1x $10^8$ pfu/ml to about 5x $10^8$ pfu/ml, within a pharmaceutically acceptable carrier. The dose is preferably about $10^5$ pfu/round to $10^{11}$ pfu/round, more preferably about $10^7$ pfu/round to $10^9$ pfu/round, and most preferably about $10^8$ pfu/round to $10^9$ pfu/round.

The compositions of the present invention comprising virus may be administered to all mammalian animals including humans, monkeys, mice, rats, rabbits, sheep, cattle, and dogs.

Brief Description of the Drawings

Fig. 1 shows the structure of the SeV vector (e/β2m/SeVb) that expresses epitope-linked-β2m (e/β2m).

Fig.2 shows the structure of the SeV vector that expresses the membrane-bound and secretory forms HLA-A*2402 molecules. A biotinylation substrate peptide (Bir A substrate peptide) is attached to the secretory form HLA-A*2402 molecules.

Fig.3 shows the structure of the SeV vector that expresses the membrane-bound form HLA-A*2402.

Fig. 4 is a graph showing the result of detection of MHC class I/peptide complex recovered from cells co-infected with SeV vectors expressing the epitope-linked-β2m and secretory form HLA-A*2402. CV-1 cells were co-infected with A24-BSP*his*/SeVb and e/β2m/Sevb at m. o. i 10 and 2, respectively. Three days later, 100 μl of the culture supernatant was collected to detect the MHC class I/peptide complex by ELISA. As negative controls, cells infected with β2m/SeVb and wt/SeV (wild-type SeV without an exogenous gene) instead of e/β2m/Sevb were used.

Fig. 5 depicts a graph and a photograph showing the purification of the secretory form MHC class I/peptide complex. CV-1 cells were co-infected with A24-BSP*his*/SeVb and e/β2m/SeVb at m.o.i 10 and 2, respectively. Three days later, 100 ml of the culture supernatant was collected and fractionated by FPLC using anti-His tag antibody. A portion of each fraction was analyzed by SDS-PAGE and Western blotting to quantify the protein concentration. About 1 mg of the secretory form MHC class I/peptide complex was recovered.

Fig. 6 is a graph showing the effect of the membrane-expressed form MHC class I/peptide complex. H9 cells (human T cell line, HLA-A*2402-) were infected with A24full/SeVb and e/Nef138-β2m/SeVb atm.o.i 10 and2, respectively. As a negative control, cells infected with wt/SeVb instead of e/Nef138-β2m/SeVb was used. Eighteen hours later, the cells were labeled with 100 μCi of $Na_2{}^{51}CrO_4$ for 2 hours to perform $^{51}Cr$ release assay using Nef-138-10-specific CTL clone.

Fig.7 shows the effect of the secretory form epitope-linked-β2m. H9 cells (HLA-A*2402-, human T cell line) were co-infected with e/Nef138-β2m/SeVb or as a control with wt/SeV at m.o.i2. Three days later, the culture supernatant was recovered and filtered using 0.22-μm membrane filter.

As target cells, H9 cells (HLA-A*2402-, human T cell line) were co-infected with A24full/SeVb and wt/SeV at m.o.i 10 and 2, respectively. Eighteen hours after the infection, the target cells were labeled with 100 μCi of $Na_2{}^{51}CrO_4$ for 2 hours. Then, the cells were pulsed with either the culture supernatant containing the e/Nef138-β2m prepared as described above, 10 μM of Nef138-10 synthetic peptide as a positive control, or the culture supernatant of the wt/SeV-infected cells as a negative control. After an hour, cells were subjected to $^{51}Cr$ release assay using Nef138-10-specific CTL clone. As a comparison, H9 cells co-infected with A24full/SeVb and e/Nef138-β2m/SeVb at m.o.i 10 and 2, respectively, were assayed similarly.

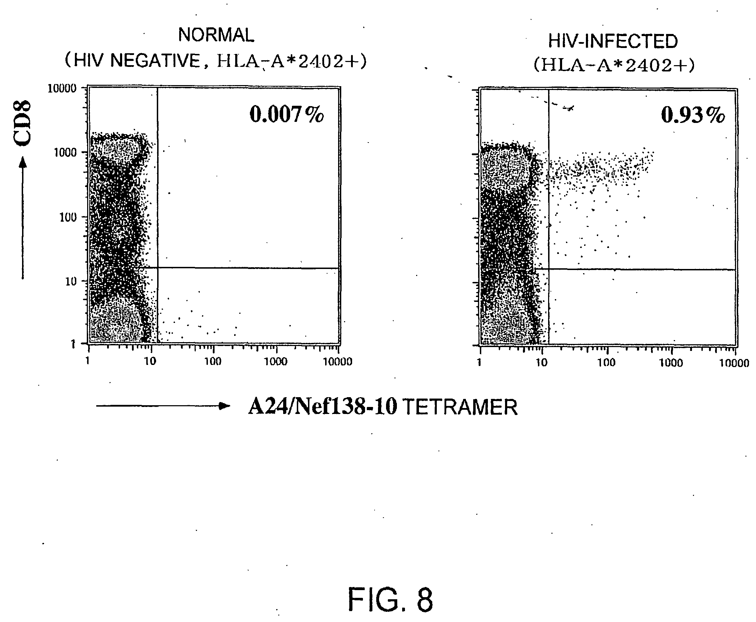

Fig.8 depicts photographs showing the detection of epitope-specific CD8-positive T cells with MHC class I/peptide tetramer. 20 μg/ml of RPE-labeled A24/Nef 138-10 tetramer, an MHC class I/peptide tetramer, was added to 1x $10^6$ of peripheral blood mononuclear cells (PBMC) from HIV-infected subject and normal healthy subject (both HLA-A*2402-positive), and incubated at 37°C for 15 min. The cells were washed once, APC-labeled anti-CD8 was added thereto and reacted at 4°C for 20 min. The cells were washed three times and fixed with PBS (phosphate-buffered saline) containing 1% paraformaldehyde. PBS containing 2% fetal calf serum and 0.1% sodium azide were used for staining and washing. A fraction containing 1x $10^5$ lymphocytes was expanded against A24/Nef138-10 tetramer and anti-CD8 antibody. The numbers in the graph indicate the percentage (%) of tetramer positive cells among CD8-positive T lymphocytes.

Best Mode for Carrying out the Invention

The present invention is specifically illustrated below with reference to the Examples, but is not to be construed as being limited thereto. Furthermore, all references cited throughout this specification are incorporated by reference.

[Example 1] Isolation of HLA-A*2402 gene and human β2m gene

The HLA-A*2402 gene, one of the human MHC class I gene, and the human β2m gene were cloned from messenger RNAs (mRNA) of peripheral blood mononuclear cells (PBMC) of a normal healthy subject carrying the HLA-A24. Micro-FastTrack Kit (Invitrogen) was used for the separation of mRNA and AMV-RT First-strand cDNA synthesis kit (LIFE SCIENCE) was used for the synthesis of cDNA.

Using the obtained cDNA as a template PCR was conducted using primer sets HLA-5P2 and HLA-3B, and b2m-5'and b2m-3' for the HLA-A*2402 and β2m genes, respectively.

```
HLA-5P2, 5'-GGGCGGATCCGGACTCAGAATCTCCCCAGACGCCGAG-3' (SEQ ID
NO: 9)
```

```
HLA-3B, 5'-CCGCCTCGAGCTGGGGAGGAAACAGGTCAGCATGGGAAC-3' (SEQ
ID NO: 10)
```

```
b2m-5', 5'-GGCACGAGCCGAGATGTCTCGCTCCGTGGC-3' (SEQ ID NO: 11)


b2m-3', 5'-AATTTGGAATTCATCCAATCCAAATGCGGC-3' (SEQ ID NO: 12)
```

PCR was carried out by 35 cycles of 94°C for 30 sec, 58°C for 30 sec, and 72°C for 1 min, followed by extension reaction at 72° for 7 min. PCR was carried out using Ex Taq (TaKaRa). PCR products thus obtained were cloned using pGEM-T vector system (Promega) (dubbed A*2402/pGEM and β2m/pGEM, respectively), and their nucleotide sequences were confirmed by sequence reactions. T.he sequence reactions were carried out using dye terminator chemistry (Big-Dye terminator cycle sequencing Ready Reaction Kit; Applied Biosystems) by electrophoresis on AB1-377 DNA Sequencer.

[Example 2] Construction of epitope-linked-β2m expression vector

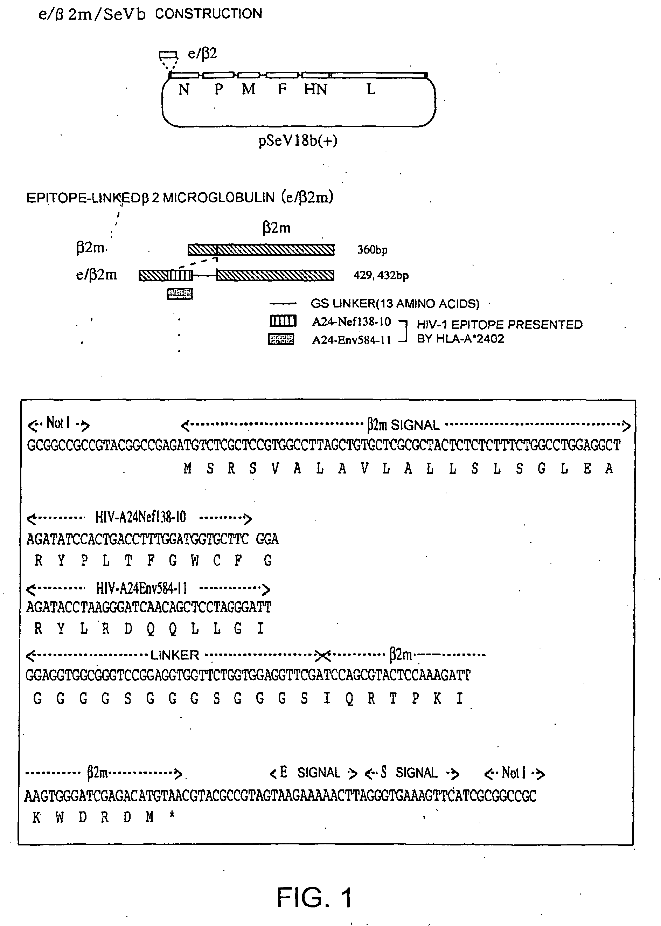

Following steps were performed to construct plasmid (e/β2m/pSeVb) that encodes a SeV vector expressing an epitope-linked- β2m. The insertion of the sequence for each epitope and linkers downstream of the β2m signal sequence, and the attachment of E and S signals of Sendai virus and *Not*I recognition site were performed by PCR (Fig.1). The amino acid sequence of the linker (GGGSGGGSGGGS/SEQ ID NO: 14) was designed to have 3 repeated sequences of GGGS (SEQ ID NO: 13).
Primers used were as follows:

```
e/b2m-a1,                                                           5'
-GGAGGTGGCGGGTCCGGAGGTGGTTCTGGTGGAGGTTCGATCCAGCGTACTCCAAAGATT-3'
(SEQ ID NO: 15)


e(Nef)-a2,                                                          5'
-TCTGGCCTGGAGGCTAGATATCCACTGACCTTTGGATGGTGCTTCGGAGGAGGTGGCGGGTCC
-3' (SEQ ID NO: 16)


e(Env)-a2',                                                         5'
-TCTGGCCTGGAGGCTAGATACCTAAGGGATCAACAGCTCCTAGGGATTGGAGGTGGCGGGTCC
-3' (SEQ ID NO: 17)


e/b2m-a3,                                                           5'
-TGCGGCCGCCGTACGGCCGAGATGTCTCGCTCCGTGGCCTTAGCTGTGCTCGCGCTACTCTCT
CTTTCTGGCCTGGAGGCT-3' (SEQ ID NO: 18)


b2m-d,                                                              5'
-TTGCGGCCGCGATGAACTTTCACCCTAAGTTTTTCTTACTACGGCGTACGTTACATGTCTCGA
TCCCACTT-3' (SEQ ID NO: 19)
```

PCR was carried out using β2m/pGEM as a template and a set of primers, e/b2m-a1 and e/b2m-d, by 15 cycles of 94°C for 1 min, 48°C for 1 min, and 72°C for 1 min, followed by an extension reaction at 72°C for 7 min. The obtained PCR product was used as a template for further PCR using e(Nef)-a2 or e(Env)-a2 and b2m-d under the same conditions, followed by another PCR using these products as a templates and e/b2m-a3 and b2m-d under the same conditions to obtain e/Nef138-β2m and e/Env584-β2m fragments . Each of these fragments was cloned using pGEM-T vector system (Promega) and their sequences were confirmed by sequence reactions. Then, each vector containing either of the fragments was digested with *Not*I and inserted into the *Not*I-digested site of pSeV18⁺b(+), and the nucleotide sequences were confirmed again. Thus, e/Nef138-β2m/pSeVb and e/Env584-β2m/pSeVb were obtained (generically dubbed as "e/β2m/pSeVb"). On the other hand, Sendei virus β2m/pSeVb expressing native β2m alone was also constructed similarly as described above, using b2m-a (5'-TGCGGCCGCCGTACGGCCGAGATGTCTCGCTCCGTGGCCTTA-3' (SEQ ID NO: 20) and b2m-d. The amino acid sequences of the Nef epitope (Nef138-10) and the Env epitope (Env584-11) are shown in SEQ ID NOs: 24 and 26, respectively.

[Example 3] Construction of vector expressing secretory form MHC class I heavy chain added with biotinylation substrate peptide

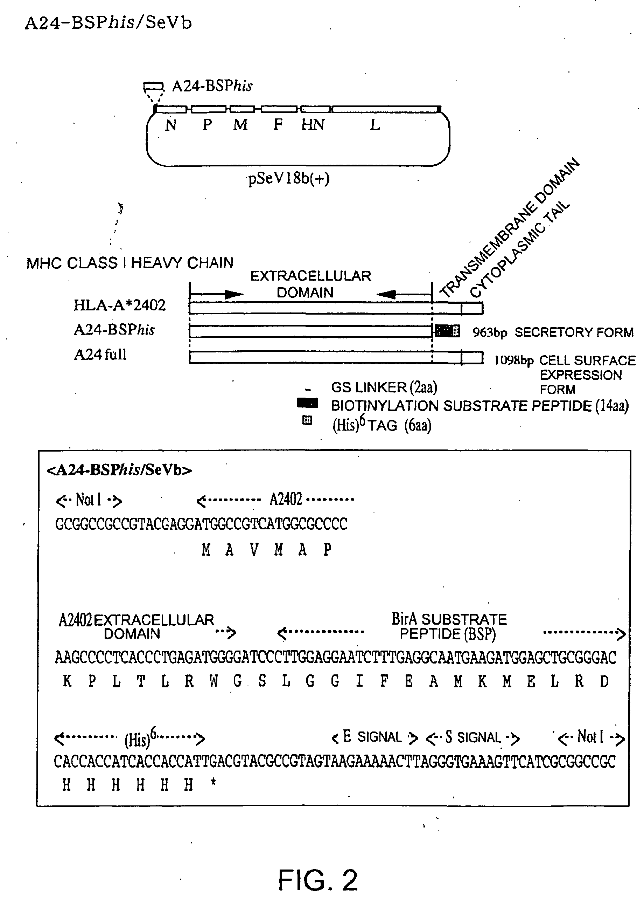

PCR was used for the attachment of Bir A substrate peptide (BSP) sequence (SEQ ID NO: 31), histidine tag (his), E and S signals of Sendai virus, and *Not* I site to the extracellular domain of HLA-A*2402 gene (Fig.2). Primers used were as follows:

```
A24-a, 5'-TGCGGCCGCCGTACGAGGATGGCCGTCATGGCGCCCCG-3' (SEQ ID NO: 32)

A24-d1,
5'-GTCCCGCAGCTCCATCTTCATTGCCTCAAAGATTCCTCCAAGGGATCCCCATCTCAGGGTG
AGGGGCTT-3' (SEQ ID NO: 33)

A24-d1/his,
5'-CTACGGCGTACGTCAATGGTGGTGATGGTGGTGGTCCCGCAGCTCCAT-3' (SEQ ID NO:
34)

A24-d2/his,
5'-TTGCGGCCGCGATGAACTTTCACCCTAAGTTTTTCTTACTACGGCGTACGTCA-3'  (SEQ
ID NO: 35)
```

PCR was carried out using A*2402/pGEM as a template and a set of primers A24-a and A24-d1, by 15 cycles of 94°C for 1 min, 48°C for 1 min, and 72°C for 1 min followed by an extension reaction at 72°C for 7 min. The PCR product was used as a template for further PCR using A24-a and A24-d1/his under the same conditions, followed by another PCR using this PCR product as a template, A24-a and A24d2/his as primers under the same conditions to obtain A24-BSP*his* fragment. A24-BSP*his*/pSeVb was obtained similarly as for the generation of e/β2m/pSeVb.

[Example 4] Construction of vector expressing membrane-bound form MHC class I heavy chain

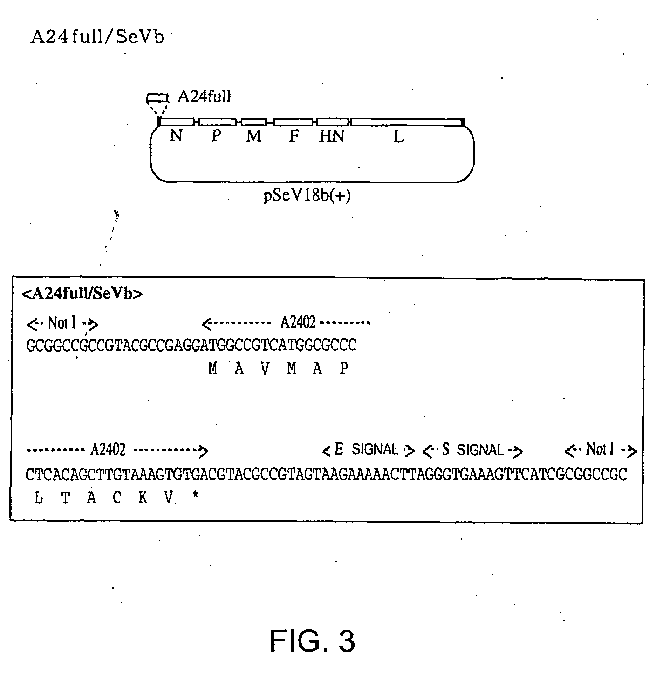

The addition of E and S signals of Sendai virus and *Not*I site were performed by PCR (Figs. 2 and 3). Primers used were as follows:

```
A24-a#, 5'-TGCGGCCGCCGTACGCCGAGGATGGCCGTCATGGCGCCCCG-3' (SEQ ID NO:
40)

A24-d4,
5'-TTGCGGCCGCGATGAACTTTCACCCTAAGTTTTTCTTACTACGGCGTACGTCACACTTTAC
AAGCTGTGAG-3' (SEQ ID NO: 41)
```

PCR was carried out using A*2402/pGEM as a template and a set of primers A24-a# and A24-d4 by 15 cycles of 94°C for 1 min, 48°C for 1 min, and 72° for 1 min, followed by an extension reaction at 72°C for 7 min, yielding A24full fragment. A24full/pSeVb was obtained similarly to the generation of e/β2m/pSeVb.

[Example'5] Reconstitution and infection of Sendai virus vector

pSeV18⁺b(+), pGEM-L, pGEM-P, pGEM-N, and vTF7-3 have been described previously (Hasan, M.K. et al., J. Gen. Virol. 78: 2813-2820 (1997); Kato, A. et al., EMBO. J. 16: 578-587 (1997); Yu, D. et al., Genes Cells, 2: 457-466 (1997)). Reconstitution of Sendai virus vector was performed according to the methods described in the above references. e/Nef138-β2m/SeVb and e/Env584-β2m/SeVb (collectively called as e/β2m/SeVb) were generated from e/ Nef138-β2m/pSeVb and e/Env584-β2m/pSeVb, respectively. Furthermore, A-24-BSP*his*/SeVb and A24fu11/SeVb were generated from A24-BSP*his*/pSeVb and A24full/pSeVb, respectively.

Simian kidney cell lines CV-1 and LLCMK2 were cultured in MEM (Sigma) supplemented with 10% fetal calf serum (FCS), 100 U/ml streptomycin, and 100 U/ml penicillin (Life Technologies) (M10). Unless otherwise stated, CV-1 cells were infected with.recombinant Sendai virus at each m.o.i and cultured in a serum-free medium for 3 days in following infection with Sendai virus.

[Example 6] Recovery and quantification of epitope-linked-β2m (e/β2m)

The culture supernatant of CV-1 cells infected with e/β2m/SeVb or β2m/SeVb was centrifuged at 40,000x g to remove Sendai virus particles and the supernatant was collected.

Sandwich enzyme-linked immunosorbent assay (ELISA) was used for the quantification of e/β2m in the culture supernatant. 2.5 µg/ml anti-human β2 microglobulin monoclonal antibody (DAKO) was used as a capture antibody, and 500 ng/ml peroxidase-labeled anti-human β2 microglobulin monoclonal antibody (DAKO) as a detector antibody. TMB peroxidase color development kit (BIO-RAD) was used for color development.

Purified human β2 microglobulin (Biogenesis) was used as the standard sample.

[Example 7] Recovery and purification of secretory form MHC class I/peptide complex

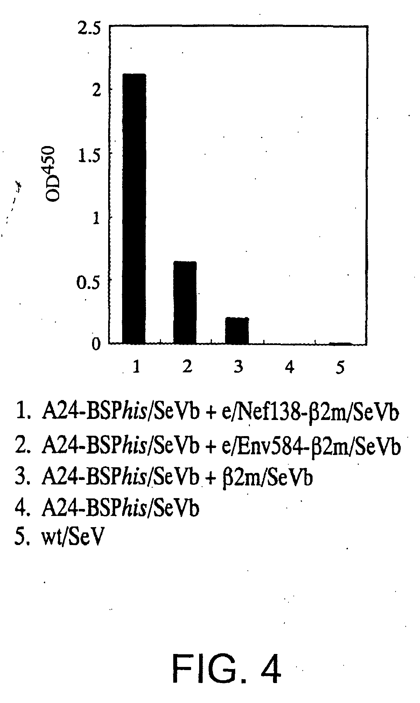

CV-1 cells were co-infected with A24-BSP*his*/SeVb and e/Nef138-β2m/SeVb or e/Env584-β2m/seVb. The culture supernatant of the infected cells was recovered, centrifuged at 40,000x g to remove Sendai virus particles and the supernatant was collected. 1mM PMSF, 3 µg/ml leupeptin, 3 µg/ml aprotinin, and 3 µg/ml pepstatin A were added thereto. The secretion of MHC class I/peptide complex into the supernatant was confirmed by sandwich ELISA. 1 µg/ ml anti-human MHC class I monoclonal antibody (Ancell) was used as a capture antibody, and 250 ng/ml peroxidase-labeled anti-human β2 microglobulin monoclonal antibody (DAKO) as a detector antibody. TMB peroxidase color development kit (BIO-RAD) was used for color development (Fig. 4) .

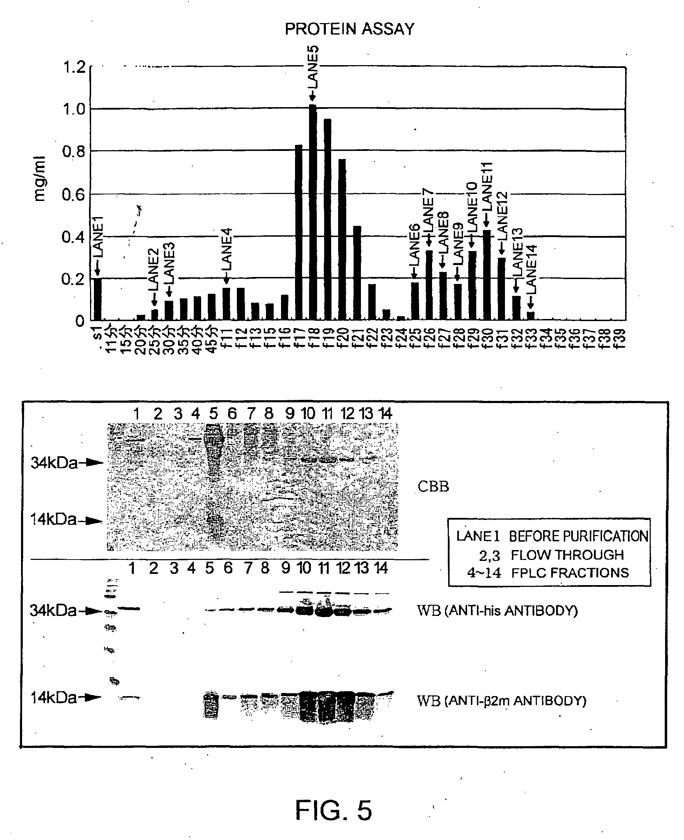

The culture supernatant was loaded onto a Hitrap-Chelating column (Amersham Pharmacia) filled with $NiSO_4$ and eluted with a gradient of 0 to 0.5 M imidazole in buffer containing 0.02 M $NaHPO_4$ and 0.5 M NaCl (pH7.4) using FPLC (Amersham Pharmacia). After substituting the elution buffer to 10 mM Tris-HCl (pH8.0) using a PD-10 column (Amersham Pharmacia), the solution was ultrafiltrated with Centricon-30 (Amicon) (Fig. 5)

[Example 8] Construction of MHC class I/peptide multimer

BirA enzyme (Avidity) was used for the addition of biotin. One mg of MHC class I/peptide complex (1.8 mg/ ml) was reacted with 10 µg BirA at 25°C for 18 hours in 10 mM Tris-HCl (pH8.0), 50 mM Bicine (pH8.3), 10 mM ATP, 10 mM MgOAc under the existence of 100 µM biotin.

The biotinylated MHC class I/peptide complex was purified on Superdex 200 HR 10/30 column (Amersham Pharmacia) with 20 mM Tris-HCl (pH8.0) -150 mM NaCl by FPLC. Then, the buffer was substituted to PBS containing 2 mM EDTA using a PD-10 column, and adjusted to 2 mg/ml by ultrafiltration using Centricon-30. The biotinylated MHC class I/peptide complex was reacted with Streptavidin-RPE (Molecular Probes) at a 1:1 ratio of the biotinylated MHC class I/peptide complex to the biotinylation site of streptavidin to form multimers.

[Example 9] Establishment of Nef 138-10-specific CTL clone

PBMC of HIV-1-infected subject carrying HLA-A*2402 was cultured overnight at 3x $10^5$/96-well in 100 µl/well R10 medium. On the next day, stimulator cells (autologous PHA-blast activated in RPMI1640 (Sigma) supplemented with 0.2 µg/ml PHA (Sigma) and 10% Lymphocult-T (Biotest) (R10) overnight, irradiated at 3000 rad, and pulsed with 10 µM Nef138-10 for 1 hour) were added and cultured for 2 weeks in the presence of 1 µg/ml anti-human CD28. The cells were further stimulated with stimulator cells (autologous Epstein-Barr virus transformed B cell line (B-LCL) irradiated at 10,000 rad and pulsed with 10 μM Nef138-10) ever 2 weeks. After 2 to 4 stimulations, when CTL activity was confirmed, the cells were cloned.

The cloning was carried out by incubating the cells at 0.8 cells/well with 1x 10⁵ cells/well stimulator cells (autologous B-LCL irradiated at 10,000 rad and pulsed with 10 μM Nef138-10) and 5x 10⁴ cells/well feeder cells (PBMC from normal healthy subject irradiated at 3,000 rad) in the presence of 10% Lymphocult-T and 2.5% PHA-sup (culture supernatant of PBMC (3x 10⁶/ml) from normal healthy subject stimulated with 0.2 μg/ml PHA for 48 hours) for 3 to 4 weeks.

[Example 10] Recognition by CTL of SeV-introduced cells forming membrane-bound form MHC class I/peptide complex

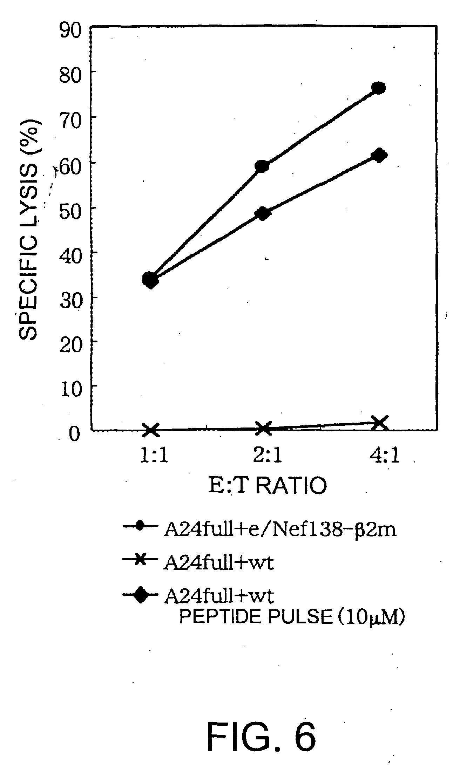

CTL $^{51}$Cr release assay was performed as follows. Human CD4$^+$ T lymphocyte cell line H9 was cultured in RPMI1160 (Sigma) supplemented with 10% FCS, 100 U/ml streptomycin, and 100 U/ml penicillin (R10). The cells (2x 10³ cells/well; target cells) were labeled with 100 μCi of Na$_2$$^{51}$CrO$_4$ for 2 hours, washed three times with R10, and pulsed with 10 μM peptide for an hour. When SeV vector-introduced cells were used as target cells, the cells were infected with SeV vectors in combinations as indicated in Fig.6 at m.o.i 10:2, 17 hours before the labeling (namely, 20 hours before the addition of the effector cells). The cells of Example 9 were added as effector cells at each E:T ratios (effector cells:target cells) indicated in Fig.6. The cells were incubated for 4 hours at 37°C, and the amount of $^{51}$Cr released in the culture supernatant was determined using a γ counter. R10 and 4% Triton X-100/PBS were added instead of the effector cells for the determination of Spontaneous release and Maximum release, respectively.

Specific lysis (%) was calculated as (cpm of each sample - cpm

of Spontaneous release) / (cpm of Maximum release - cpm of Spontaneous

release) x 100.

Fig.6 shows the result of recognition by CTL of the membrane-bound form MHC class I/peptide complexes. It was revealed that antigen-specific CTL activation could be detected in the cells co-infected with A24full/SeVb and e/Nef138-β2m/SeVb.

[Example 11] Effect of epitope-linked-β2m recovered from SeV-infected cells

In order to prepare epitope-linked-β2m produced by SeV-infected cells, H9 cells were infected with e/Nef138-β2m/SeVb at m.o.i 2. Three days later, culture supernatant was collected and filtered, yielding a solution containing the epitope-linked-β2m. Similarly as in Example 10, CTL assay was carried out using H9 cells infected with A24full/SeVb as target cells to compare the effect of pulsing with peptides and that with the above-obtained epitope-linked-β2m-containing solution.

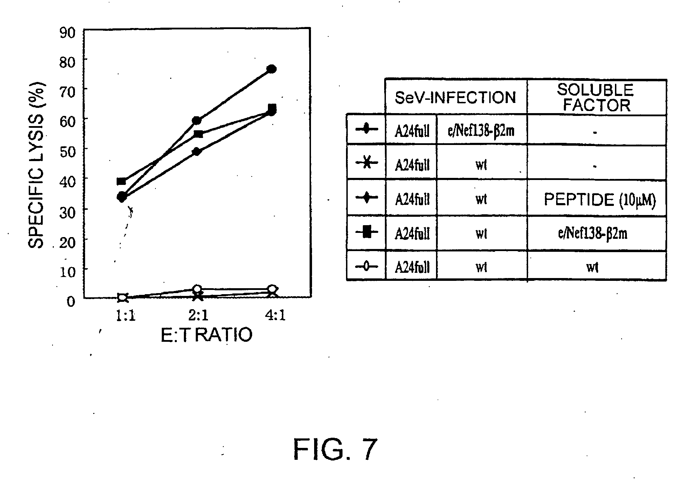

Fig.7 shows the effect of epitope-linked-β2m. Antigen-specific CTL activity was revealed to be detected upon the addition of the epitope-linked-β2m recovered from the culture supernatant of SeV-infected cells to the culture solution of the target cells.

[Example 12] Detection of epitope-specific CD8-positive T cells by MHC class I/peptide tetramer

20 μg/ml RPE-labeled A24/Nef138-10 tetramer, an MHC class I/peptide tetramer, was added to 1 x 10⁶ peripheral blood mononuclear cells (PBMC) from HIV-1-infected and normal healthy subjects (both carrying HLA-A*2402), and reacted at 37°C for 15 min. The cells were washed once, APC-labeled anti-CD8 was added thereto and reacted at 4°C for 20 min. After washing three times, cells were fixed with PBS (phosphate-buffered saline) containing 1% paraformaldehyde. PBS containing 2% FCS and 0.1% sodium azide was used for staining and washing. The results are shown in Fig.8. In the figure, 1x 10⁵ lymphocyte fraction was expanded against A24/Nef138-10 tetramer and anti-CD8, and the numbers indicate the percentage (%) of tetramer-positive T cells among the CD8-positive T cells.

Industrial Applicability

According to the present invention, epitope-linked-β2m and MHC class I/peptide complexes can be efficiently expressed in mammalian cells. The epitope-linked-β2m and MHC class I/peptide complexes thus obtained are extremely useful for: (1) the detection and quantification of antigen-specific CTL; (2) antigen presentation to antigen-specific CTL utilizing the purified epitope-linked-β2m protein; and (3) induction of antigen-specific cellular immune response via *in vivo* or *ex vivo* introduction of a virus vector. The epitope-linked-β2m and MHC class I/peptide complexes that can be obtained by the present invention are useful for the detection and quantification of CTL reactive to autologous antigens, virus antigens, tumor antigens, or the like. In addition, these complexes are useful for the induction of protective immunity against infection with viruses, bacteria, etc., and for immunotherapy against cancers. Moreover, the vectors of the present invention are useful as vectors for gene therapy in the induction of protective immunity for infectious diseases and immunotherapy for cancers.

SEQUENCE LISTING

<110> DNAVEC RESEARCH INC.

<120> MAMMALIAN CELL-INFECTING VIRUS VECTOR ENCODING EPITOPE-LINKED-$\beta$2m AND USES THEREOF

<130> D3-A0101P

<140>

<141>

<150> JP    2001-301290

<151> 2001-09-28

<160> 45

<170> PatentIn Ver. 2.0

<210> 1

<211> 10

<212> DNA

<213> Artificial Sequence

<220>

<223> Description of Artificial Sequence: artificially synthesized sequence

<400> 1

ctttcaccct                                                                10

<210> 2

<211> 15

<212> DNA

<213> Artificial Sequence

<220>

<223> Description of Artificial Sequence: artificially
    synthesized sequence


<400> 2
tttttcttac tacgg                                                    15



<210> 3
<211> 18
<212> DNA
<213> Artificial Sequence


<220>

<223> Description of Artificial Sequence: artificially
    synthesized sequence


<400> 3
cggccgcaga tcttcacg                                                 18



<210> 4
<211> 18
<212> DNA
<213> Artificial Sequence


<220>

<223> Description of Artificial Sequence: artificially
    synthesized sequence


<400> 4
atgcatgccg gcagatga                                                 18

    ---


<210> 5

<211> 18

<212> DNA

<213> Artificial Sequence

<220>

<223> Description of Artificial Sequence: artificially
synthesized sequence

<400> 5

gttgagtact gcaagagc                                                    18


<210> 6

<211> 42

<212> DNA

<213> Artificial Sequence

<220>

<223> Description of Artificial Sequence: artificially
synthesized sequence

<400> 6

tttgccggca tgcatgtttc ccaaggggag agttttgcaa cc                          42


<210> 7

<211> 18

<212> DNA

<213> Artificial Sequence

<220>

<223> Description of Artificial Sequence: artificially
synthesized sequence

<400> 7

atgcatgccg gcagatga                                                    18

<210> 8
<211> 21
<212> DNA
<213> Artificial Sequence

<220>
<223> Description of Artificial Sequence: artificially
      synthesized sequence

<400> 8
tgggtgaatg agagaatcag c                                    21


<210> 9
<211> 37
<212> DNA
<213> Artificial Sequence

<220>
<223> Description of Artificial Sequence: artificially
      synthesized sequence

<400> 9
gggcggatcc ggactcagaa tctccccaga cgccgag                   37


<210> 10
<211> 39
<212> DNA
<213> Artificial Sequence

<220>
<223> Description of Artificial Sequence: artificially
      synthesized sequence

<400> 10
ccgcctcgag ctggggagga aacaggtcag catgggaac          39


<210> 11
<211> 30
<212> DNA
<213> Artificial Sequence

<220>
<223> Description of Artificial Sequence: artificially
      synthesized sequence

<400> 11
ggcacgagcc gagatgtctc gctccgtggc          30


<210> 12
<211> 30
<212> DNA
<213> Artificial Sequence

<220>
<223> Description of Artificial Sequence: artificially
      synthesized sequence

<400> 12
aatttggaat tcatccaatc caaatgcggc          30


<210> 13
<211> 4
<212> PRT
<213> Artificial Sequence

<220>

<223> Description of Artificial Sequence: artificially
      synthesized sequence

<400> 13

Gly Gly Gly Ser
  1

<210> 14
<211> 12
<212> PRT
<213> Artificial Sequence

<220>

<223> Description of Artificial Sequence: artificially
      synthesized sequence

<400> 14

Gly Gly Gly Ser Gly Gly Gly Ser Gly Gly Gly Ser
   1               5                   10

<210> 15
<211> 60
<212> DNA
<213> Artificial Sequence

<220>

<223> Description of Artificial Sequence: artificially
      synthesized sequence

<400> 15

ggaggtggcg ggtccggagg tggttctggt ggaggttcga tccagcgtac tccaaagatt 60

\<210\> 16

\<211\> 63

\<212\> DNA

\<213\> Artificial Sequence

\<220\>

\<223\> Description of Artificial Sequence: artificially
synthesized sequence

\<400\> 16

tctggcctgg aggctagata tccactgacc tttggatggt gcttcggagg aggtggcggg 60

tcc                                                                    63

\<210\> 17

\<211\> 63

\<212\> DNA

\<213\> Artificial Sequence

\<220\>

\<223\> Description of Artificial Sequence: artificially
synthesized sequence

\<400\> 17

tctggcctgg aggctagata cctaagggat caacagctcc tagggattgg aggtggcggg 60

tcc                                                                    63

\<210\> 18

\<211\> 81

\<212\> DNA

\<213\> Artificial Sequence

\<220\>

<223> Description of Artificial Sequence: artificially
synthesized sequence

<400> 18
tgcggccgcc gtacggccga gatgtctcgc tccgtggcct tagctgtgct cgcgctactc 60

tctctttctg gcctggaggc t                                                81

<210> 19
<211> 71
<212> DNA
<213> Artificial Sequence

<220>
<223> Description of Artificial Sequence: artificially
synthesized sequence

<400> 19
ttgcggccgc gatgaacttt caccctaagt ttttcttact acggcgtacg ttacatgtct 60

cgatcccact t                                                71

<210> 20
<211> 42
<212> DNA
<213> Artificial Sequence

<220>
<223> Description of Artificial Sequence: artificially
synthesized sequence

<400> 20
tgcggccgcc gtacggccga gatgtctcgc tccgtggcct ta                42

<210> 21
<211> 80
<212> DNA
<213> Artificial Sequence

<220>
<223> Description of Artificial Sequence: artificially
synthesized sequence

<220>
<221> CDS
<222> (21)..(80)

<400> 21
gcggccgccg tacggccgag atg tct cgc tcc gtg gcc tta gct gtg ctc gcg 53
                      Met Ser Arg Ser Val Ala Leu Ala Val Leu Ala
                       1               5                  10

cta ctc tct ctt tct ggc ctg gag gct                              80
Leu Leu Ser Leu Ser Gly Leu Glu Ala
             15                  20


<210> 22
<211> 20
<212> PRT
<213> Artificial Sequence

<400> 22
Met Ser Arg Ser Val Ala Leu Ala Val Leu Ala Leu Leu Ser Leu Ser
 1               5                  10                  15

Gly Leu Glu Ala
             20

<210> 23
<211> 30
<212> DNA
<213> Artificial Sequence

<220>
<223> Description of Artificial Sequence: artificially
      synthesized sequence

<220>
<221> CDS
<222> (1)..(30)

<400> 23
aga tat cca ctg acc ttt gga tgg tgc ttc                    30
Arg Tyr Pro Leu Thr Phe Gly Trp Cys Phe
    1               5                   10


<210> 24
<211> 10
<212> PRT
<213> Artificial Sequence

<400> 24
Arg Tyr Pro Leu Thr Phe Gly Trp Cys Phe
    1               5                   10


<210> 25
<211> 33
<212> DNA
<213> Artificial Sequence

<220>

<223> Description of Artificial Sequence: artificially
         synthesized sequence

<220>
<221> CDS
<222> (1)..(33)

<400> 25
aga tac cta agg gat caa cag ctc cta ggg att                    33
Arg Tyr Leu Arg Asp Gln Gln Leu Leu Gly Ile
  1               5                   10


<210> 26
<211> 11
<212> PRT
<213> Artificial Sequence

<400> 26
Arg Tyr Leu Arg Asp Gln Gln Leu Leu Gly Ile
  1               5                   10


<210> 27
<211> 60
<212> DNA
<213> Artificial Sequence

<220>
<223> Description of Artificial Sequence: artificially
         synthesized sequence

<220>
<221> CDS
<222> (1)..(60)

<400> 27

```
gga ggt ggc ggg tcc gga ggt ggt tct ggt gga ggt tcg atc cag cgt   48
Gly Gly Gly Gly Ser Gly Gly Gly Ser Gly Gly Gly Ser Ile Gln Arg
 1               5                   10                  15

act cca aag att                                                    60
Thr Pro Lys Ile
                20
```

<210> 28
<211> 20
<212> PRT
<213> Artificial Sequence

<400> 28

```
Gly Gly Gly Gly Ser Gly Gly Gly Ser Gly Gly Gly Ser Ile Gln Arg
 1               5                   10                  15

Thr Pro Lys Ile
                20
```

<210> 29
<211> 69
<212> DNA
<213> Artificial Sequence

<220>
<223> Description of Artificial Sequence: artificially
      synthesized sequence

<220>
<221> CDS
<222> (1)..(18)

<400> 29

aag tgg gat cga gac atg taacgtacgc cgtagtaaga aaaacttagg          48
Lys Trp Asp Arg Asp Met
  1               5


gtgaaagttc atcgcggccg c          69


<210> 30
<211> 6
<212> PRT
<213> Artificial Sequence


<400> 30
Lys Trp Asp Arg Asp Met
  1               5


<210> 31
<211> 14
<212> PRT
<213> Artificial Sequence


<220>
<223> Description of Artificial Sequence: artificially
      synthesized sequence


<400> 31
Leu Gly Gly Ile Phe Glu Ala Met Lys Met Glu Leu Arg Asp
  1               5               10


<210> 32
<211> 38
<212> DNA
<213> Artificial Sequence

<220>
<223> Description of Artificial Sequence: artificially
     synthesized sequence

<400> 32
tgcggccgcc gtacgaggat ggccgtcatg gcgccccg           38

<210> 33
<211> 69
<212> DNA
<213> Artificial Sequence

<220>
<223> Description of Artificial Sequence: artificially
     synthesized sequence

<400> 33
gtcccgcagc tccatcttca ttgcctcaaa gattcctcca agggatcccc atctcagggt  60

gaggggctt                                69

<210> 34
<211> 48
<212> DNA
<213> Artificial Sequence

<220>
<223> Description of Artificial Sequence: artificially
     synthesized sequence

<400> 34
ctacggcgta cgtcaatggt ggtgatggtg gtggtcccgc agctccat         48

<210> 35

<211> 53

<212> DNA

<213> Artificial Sequence


<220>

<223> Description of Artificial Sequence: artificially
        synthesized sequence


<400> 35

ttgcggccgc gatgaacttt caccctaagt ttttcttact acggcgtacg tca                53



<210> 36

<211> 36

<212> DNA

<213> Artificial Sequence


<220>

<223> Description of Artificial Sequence: artificially
        synthesized sequence


<220>

<221> CDS

<222> (18)..(36)


<400> 36

gcggccgccg tacgagg atg gcc gtc atg gcg ccc c                                36
                        Met Ala Val Met Ala Pro
                         1               5



<210> 37

<211> 6

<212> PRT

<213> Artificial Sequence

<400> 37

Met Ala Val Met Ala Pro
1           5

<210> 38

<211> 138

<212> DNA

<213> Artificial Sequence

<220>

<223> Description of Artificial Sequence: artificially
      synthesized sequence

<220>

<221> CDS

<222> (1)..(87)

<400> 38

aag ccc ctc acc ctg aga tgg gga tcc ctt gga gga atc ttt gag gca      48
Lys Pro Leu Thr Leu Arg Trp Gly Ser Leu Gly Gly Ile Phe Glu Ala
  1           5               10                  15

atg aag atg gag ctg cgg gac cac cac cat cac cac cat tgacgtacgc      97
Met Lys Met Glu Leu Arg Asp His His His His His His
              20                  25

cgtagtaaga aaaacttagg gtgaaagttc atcgcggccg c                       138

<210> 39

<211> 29

<212> PRT

<213> Artificial Sequence

<400> 39

Lys Pro Leu Thr Leu Arg Trp Gly Ser Leu Gly Gly Ile Phe Glu Ala
1               5                   10                  15

Met Lys Met Glu Leu Arg Asp His His His His His His
            20                  25

<210> 40
<211> 41
<212> DNA
<213> Artificial Sequence

<220>
<223> Description of Artificial Sequence: artificially
      synthesized sequence

<400> 40
tgcggccgcc gtacgccgag gatggccgtc atggcgcccc g                          41

<210> 41
<211> 71
<212> DNA
<213> Artificial Sequence

<220>
<223> Description of Artificial Sequence: artificially
      synthesized sequence

<400> 41
ttgcggccgc gatgaacttt caccctaagt ttttcttact acggcgtacg tcacacttta 60

caagctgtga g                                                           71

<210> 42
<211> 38
<212> DNA
<213> Artificial Sequence

<220>
<223> Description of Artificial Sequence: artificially
synthesized sequence

<220>
<221> CDS
<222> (21)..(38)

<400> 42
gcggccgccg tacgccgagg atg gcc gtc atg gcg ccc                                    38
                         Met Ala Val Met Ala Pro
                          1               5

<210> 43
<211> 6
<212> PRT
<213> Artificial Sequence

<400> 43
Met Ala Val Met Ala Pro
 1               5

<210> 44
<211> 69
<212> DNA
<213> Artificial Sequence

<220>

```
<223> Description of Artificial Sequence: artificially
       synthesized sequence


<220>
<221> CDS
<222> (1)..(18)


<400> 44
ctc aca gct tgt aaa gtg tgacgtacgc cgtagtaaga aaaacttagg        48
Leu Thr Ala Cys Lys Val
  1               5


gtgaaagttc atcgcggccg c                                         69



<210> 45
<211> 6
<212> PRT
<213> Artificial Sequence


<400> 45
Leu Thr Ala Cys Lys Val
  1               5
```

**Claims**

1. A mammalian cell-infecting virus vector that encodes an epitope-linked-β2m in an expressible manner.

2. The virus vector of claim 1, wherein said mammalian cell-infecting virus vector is a Paramyxovirus vector.

3. The virus vector of claim 2, wherein said Paramyxovirus is Sendai virus.

4. The virus vector of any one of claims 1 to 3, wherein said epitope comprises an amino acid sequence of an epitope peptide presented by an antigen-presenting cell, or a portion thereof.

5. The virus vector of any one of claims 1 to 4, wherein said epitope is a partial peptide of a HIV-1 virus protein.

6. The virus vector of claim 5, wherein said epitope comprises the amino acid sequence of SEQ ID NO: 24 or 26, or a portion thereof.

7. The virus vector of any one of claims 1 to 4, wherein said epitope is a partial peptide of a tumor antigen.

8. The virus vector of any one of claims 1 to 7, wherein said vector comprises the amino acid sequence of SEQ ID NO: 13 or a repeated sequence thereof between said epitope and β2m sequences.

9. A method for producing an epitope-linked-β2m, which comprises the steps of:

    (a) introducing the virus vector of any one of claims 1 to 8 into a mammalian cell, and
    (b) recovering the epitope-linked-β2m from the mammalian cell introduced with the vector or the culture supernatant thereof.

10. An epitope-linked-β2m produced by the method of claim 9.

11. A method for producing a heterodimer comprising an epitope-linked-β2m, which comprises the steps of:

    (a) introducing the virus vector of any one of claims 1 to 8 into a mammalian cell, and
    (b) recovering the produced heterodimer from the mammalian cell introduced with the vector or the culture supernatant thereof.

12. A method for producing an MHC class I/peptide complex comprising an MHC class I heavy chain and an epitope-linked-β2m, which comprises the steps of:

    (a) introducing the virus vector of any one of claims 1 to 8 into a mammalian cell, and
    (b) recovering the produced MHC class I/peptide complex from the mammalian cell introduced with the vector or the culture supernatant thereof.

13. The method of claim 12, wherein said method further comprises the step of introducing a virus vector expressing the MHC class I heavy chain into the mammalian cell.

14. The method of claim 13, wherein said MHC class I heavy chain is a secretory form.

15. The method of claim 14, wherein said MHC class I heavy chain comprises a biotinylation substrate peptide.

16. The method of any one of claims 13 to 15, wherein said MHC class I heavy chain is an A24-restricted HLA class I heavy chain.

17. The method of claim 16, wherein said A24-restricted HLA class I heavy chain is derived from A*2402.

18. The method of claim 15, wherein said method further comprises the steps of biotinylating said MHC class I heavy chain comprising the biotinylation substrate peptide and assembling the biotinylated MHC class I heavy chain in the presence of avidin.

19. An MHC class I/peptide complex produced by the method of any one of claims 12 to 18.

20. The MHC class I/peptide complex of claim 19, wherein said complex is a tetramer.

21. An inducer of antigen-specific cellular immune response comprising the virus vector of any one of claims 1 to 8, the epitope-linked-β2m of claim 10, or the MHC class I/peptide complex of claim 19 or 20.

22. A pharmaceutical composition comprising the virus vector of any one of claims 1 to 8, the epitope-linked-β2m of claim 10, or the MHC class I/peptide complex of claim 19 or 20.

23. A mammalian cell, wherein the virus vector of any one of claims 1 to 8 has been introduced.

24. A cell that has the epitope-linked-β2m of claim 10 on the cell surface.

25. The cell of claim 23 or 24, wherein a gene encoding an MHC class I heavy chain has been exogenously introduced.

26. The cell of claim 25, wherein said MHC class I heavy chain is a membrane-bound form.

**27.** The cell of claim 25, wherein said MHC class I heavy chain is a secretory form.

**28.** The cell of any one of claims 23 to 27, which is a dendritic cell.

**29.** An inducer of antigen-specific cellular immune response comprising the cell of any one of claims 23 to 28.

**30.** A pharmaceutical composition comprising the cell of any one of claims 23 to 28.

**31.** A detection agent for antigen-specific T lymphocytes comprising the MHC class I/peptide complex of claim 19 or 20.

e/β 2m/SeVb CONSTRUCTION

```
        ⊏⊐ e/β2
   ┌──────────────────────────────────┐
   │ N   P   M   F   HN      L         │
   └──────────────────────────────────┘
```

pSeV18b(+)

EPITOPE-LINKED β 2 MICROGLOBULIN (e/β2m)

β2m

β2m.        ▨▨▨▨▨▨▨▨▨▨▨▨        360bp

e/β2m       ▨▨▨▨▨⫿⫿⫿⫿——▨▨▨▨▨▨▨▨▨▨        429, 432bp
            ▨▨▨▨

—— GS LINKER(13 AMINO ACIDS)

⫿⫿⫿ A24-Nef138-10 ⎤ HIV-1 EPITOPE PRESENTED
▨▨▨ A24-Env584-11 ⎦ BY HLA-A*2402

---

<·· Not I ·>                    <·············· β2m SIGNAL ··············>
GCGGCCGCCGTACGGCCGAGATGTCTCGCTCCGTGGCCTTAGCTGTGCTCGCGCTACTCTCTCTTTCTGGCCTGGAGGCT
                      M   S   R   S   V   A   L   A   V   L   A   L   L   S   L   S   G   L   E   A


<········ HIV-A24Nef138-10 ········>
AGATATCCACTGACCTTTGGATGGTGCTTC GGA
 R   Y   P   L   T   F   G   W   C   F   G

<········ HIV-A24Env584-11 ···········>
AGATACCTAAGGGATCAACAGCTCCTAGGGATT
 R   Y   L   R   D   Q   Q   L   L   G   I

<·············· LINKER ·············>✕······· β2m ···········
GGAGGTGGCGGGTCCGGAGGTGGTTCTGGTGGAGGTTCGATCCAGCGTACTCCAAAGATT
 G   G   G   G   S   G   G   G   S   G   G   G   S   I   Q   R   T   P   K   I


········· β2m···········>        <E SIGNAL > <··S SIGNAL ·>   <·· Not I ·>
AAGTGGGATCGAGACATGTAACGTACGCCGTAGTAAGAAAAACTTAGGGTGAAAGTTCATCGCGGCCGC
 K   W   D   R   D   M   *
```

FIG. 1

A24-BSP*his*/SeVb

FIG. 2

A24full/SeVb

A24full

N    P    M    F    HN    L

pSeV18b(+)

<A24full/SeVb>

<- Not I ->          <--------- A2402 --------->
GCGGCCGCCGTACGCCGAGGATGGCCGTCATGGCGCCC
                           M  A  V  M  A  P

---------- A2402 ----------->          < E SIGNAL > <- S SIGNAL ->       <- Not I ->
CTCACAGCTTGTAAAGTGTGACGTACGCCGTAGTAAGAAAAACTTAGGGTGAAAGTTCATCGCGGCCGC
  L  T  A  C  K  V  *

FIG. 3

1. A24-BSP*his*/SeVb + e/Nef138-β2m/SeVb
2. A24-BSP*his*/SeVb + e/Env584-β2m/SeVb
3. A24-BSP*his*/SeVb + β2m/SeVb
4. A24-BSP*his*/SeVb
5. wt/SeV

# FIG. 4

FIG. 5

FIG. 6

| | SeV-INFECTION | | SOLUBLE FACTOR |
|---|---|---|---|
| ◆ | A24full | e/Nef138-β2m | - |
| ✳ | A24full | wt | - |
| ◆ | A24full | wt | PEPTIDE (10μM) |
| ■ | A24full | wt | e/Nef138-β2m |
| ○ | A24full | wt | wt |

FIG. 7

FIG. 8

<table>
<tr><td colspan="2">**INTERNATIONAL SEARCH REPORT**</td><td>International application No.<br>PCT/JP02/09697</td></tr>
</table>

| A. CLASSIFICATION OF SUBJECT MATTER |
| --- |
| Int.Cl⁷ C12N15/86, C12P21/02, C07K14/74, C12N5/00, A61K45/00,<br>A61K48/00, A61P31/00, A61P35/00 |

According to International Patent Classification (IPC) or to both national classification and IPC

| B. FIELDS SEARCHED |
| --- |
| Minimum documentation searched (classification system followed by classification symbols)<br>Int.Cl⁷ C12N15/86, C12P21/02, C07K14/74, C12N5/00, A61K45/00,<br>A61K48/00, A61P31/00, A61P35/00 |

Documentation searched other than minimum documentation to the extent that such documents are included in the fields searched

Electronic data base consulted during the international search (name of data base and, where practicable, search terms used)
CAPLUS/MEDLINE/BIOSIS/WPIDS(STN), SwisssProt/PIR/Geneseq

| C. DOCUMENTS CONSIDERED TO BE RELEVANT | | |
| --- | --- | --- |
| Category* | Citation of document, with indication, where appropriate, of the relevant passages | Relevant to claim No. |
| X<br><br>Y | Tafuro S. et al., "Reconstitution of antigen presentation in HLA class I-negative cancer calls with peptide-beta2m fusion molecules", Eur. J. Immunol., 2001 Feb., Vol.31, No.2, pages 440 to 449, particularly, page 442, left column, Par. No. [0002]; Fig. 1B, and "Discussion" | 1,4,7,8-15,<br>19,21-31<br>2,3,5,6,<br>16-18,20 |
| X<br>Y | WO 94/24290 A1 (BRITISH BIO-TECHNOLOGY LTD.),<br>27 October, 1994 (27.10.94),<br>Particularly, Claim 8 and example 8<br>& AU 9464353 A1 & EP 693125 A1<br>& US 2002/123108 A1 | 19,21-31<br>1-18,20 |

| ☒ | Further documents are listed in the continuation of Box C. | ☐ | See patent family annex. |
| --- | --- | --- | --- |

| * Special categories of cited documents: | "T" later document published after the international filing date or |
| --- | --- |
| "A" document defining the general state of the art which is not considered to be of particular relevance | priority date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such |
| "O" document referring to an oral disclosure, use, exhibition or other means | combination being obvious to a person skilled in the art |
| "P" document published prior to the international filing date but later than the priority date claimed | "&" document member of the same patent family |

| Date of the actual completion of the international search<br>13 November, 2002 (13.11.02) | Date of mailing of the international search report<br>26 November, 2002 (26.11.02) |
| --- | --- |
| Name and mailing address of the ISA/<br>Japanese Patent Office | Authorized officer |
| Facsimile No. | Telephone No. |

Form PCT/ISA/210 (second sheet) (July 1998)

**EP 1 447 451 A1**

**INTERNATIONAL SEARCH REPORT**

International application No.

PCT/JP02/09697

C (Continuation).  DOCUMENTS CONSIDERED TO BE RELEVANT

| Category* | Citation of document, with indication, where appropriate, of the relevant passages | Relevant to claim No. |
|---|---|---|
| X<br>Y | Uger, R.A. et al., "Covalent linkage to beta 2-microglobulin enhances the MHC stability and antigenicity of suboptimal CTL epitopes", J. Immunol., 1999, Vol.162, No.10, pages 6024 to 6028, Figs. 2 to 4 | 19,21-31<br>1-18,20 |
| X<br>Y | Uger, R.A & Barber, B.H., "Creating CTL targets with epitope-linked beta 2-microglobulin constructs", J. Immunol., 1998, Vol.160, No.4, pages 1598 to 1605, Figs. 1, 3 | 19,21-31<br>1-18,20 |
| X<br>Y | White, J., et al., "Soluble class I MHC with beta2-microglobulin covalently linked peptides: specific binding to a T cell hybridoma" J. Immunol., 1999, Vol.162, No.5, pages 2671 to 2676, Fig. 1 | 19,21-31<br>1-18,20 |
| X<br>Y | Toshitani, K., et al., "Expression of a single-chain HLA class I molecule in a human cell line: presentation of exogenous peptide and processesd antigen cytotoxic T lymphocytes", Proc. Natl. Acad. Sci. USA, 1996, Vol.93, No.1, pages 236 to 240, "Materials and Methods" | 19,21-31<br>1-18,20 |
| Y | Kato, A., et al., "The paramyxovirus, Sendai Virus, V protein encodes a luxury function reqired for viral pathogenesis", EMBO J., 1997, Vol.16, No.3, pages 578 to 587, "Discussion" | 1-31 |
| Y | WO 01/18223 A1 (DNAVEC RESEARCH INC.), 15 March, 2001 (15.03.01), Claims; abstract & AU 2000068720 A & EP 1211318 A1 & KR 2002013565 A | 1-31 |
| Y | Ramani, K., et al., "Site-specific gene-delivery in vivo through engineered Sendai viral envelopes" Proc. Natl. Acad. Sci. USA, 1998, Vol.95, pages 11886 to 11890, abstract | 1-31 |
| Y | WO 01/57204 A1 (KANEDA, Yasufumi), 09 August, 2001 (09.08.01), Abstract & JP 2001-286282 A & EP 1170363 A1 | 1-31 |
| Y | WO 01/24810 A1 (EPIMMUNE INC.), 12 April, 2001 (12.04.01), pages 186, 362, 192, 364 & AU 2001075001 A | 6 |

Form PCT/ISA/210 (continuation of second sheet) (July 1998)